# EUROPEAN PATENT SPECIFICATION

(11) **EP 4 393 384 B1**
(45) Date of publication and mention of the grant of the patent: **26.08.2026**
(21) Application number: 22910101.9
(22) Date of filing: 21.12.2022
(51) Int. Cl.: A61B 5/022, A61B 5/0225, A61B 5/00, A61B 5/021

(54) **BLOOD PRESSURE MEASURING DEVICE AND ELECTRONIC DEVICE**
BLUTDRUCKMESSVORRICHTUNG UND ELEKTRONISCHE VORRICHTUNG
DISPOSITIF DE MESURE DE PRESSION ARTÉRIELLE ET DISPOSITIF ÉLECTRONIQUE

(30) Priority: 23.12.2021 CN 202111591753
(43) Date of publication of application: 03.07.2024
(73) Proprietor: Huawei Technologies Co., Ltd., Shenzhen, Guangdong 518129 (CN)
(72) Inventor: JIN, Junye, Shenzhen, Guangdong 518129 (CN); YANG, Sulin, Shenzhen, Guangdong 518129 (CN); ZENG, Zhao, Shenzhen, Guangdong 518129 (CN); ZHANG, Lei, Shenzhen, Guangdong 518129 (CN); LIN, Zhongwei, Shenzhen, Guangdong 518129 (CN)
(74) Representative: Maiwald GmbH
(86) International application number: PCT/CN2022/140802
(87) International publication number: WO 2023/116789

(56) References cited:
- CN-A- 103 025 231
- CN-A- 106 793 963
- GB-A- 2 541 368
- JP-A- 2009 153 843
- JP-A- 2010 131 247
- JP-A- 2012 205 719
- US-B1- 10 849 555

## Description

### CROSS-REFERENCE TO RELATED APPLICATIONS

This application claims priority to Chinese Patent Application No. 202111591753.0, filed with the China National Intellectual Property Administration on December 23, 2021 and entitled "BLOOD PRESSURE MEASUREMENT DEVICE AND ELECTRONIC DEVICE".

### TECHNICAL FIELD

This application relates to the field of electronic device technologies, and in particular, to a blood pressure measurement device and an electronic device.

### BACKGROUND

Nowadays, people pay more attention to health conditions of themselves and their families, and blood pressure measurement is especially important. With progress and development of science and technology, a blood pressure measurement device for home use appears, and a blood pressure measurement function is integrated into some wearable devices (such as a smart watch or a smart band), and this provides a possibility for a user to perform blood pressure measurement anytime and anywhere.

In a current blood pressure measurement device, a micro pump and a pressure sensor are directly placed inside a body of the blood pressure measurement device. The pressure sensor may obtain a blood pressure value of a user by measuring air pressure of the blood pressure measurement device. However, in a process in which the blood pressure measurement device is applied to perform blood pressure measurement, because the micro pump inflates an airbag with air inside the body, an air flow is generated in an inflating process, and the air flow causes fluctuation of air pressure. Consequently, air pressure detected by the pressure sensor is unstable, and accuracy of a blood pressure value measured by the blood pressure measurement device is affected.

Therefore, how to provide a blood pressure measurement device that can satisfy blood pressure measurement accuracy has become an urgent difficult problem to be resolved by a person skilled in the art.
Document JP 2010 131247 A discloses a blood pressure measuring apparatus. Document CN 106 793 963 A discloses a method for oscillatory non-invasive blood pressure (nibp) measurement and control unit for an nibp apparatus. Document CN 103 025 231 A discloses an electronic blood pressure sphygmomanometer. Document GB 2 541 368 A discloses a fluid delivery apparatus. Document JP 2009 153843 A discloses a blood pressure measuring apparatus. Document JP 2012 205719 A discloses a sphygmomanometer. Document US 10 849 555 B1 discloses devices and systems for correcting errors in blood pressure measurements.

### SUMMARY

This application provides a blood pressure measurement device and an electronic device, to reduce impact of internal air pressure of the blood pressure measurement device on blood pressure measurement of the blood pressure measurement device, thereby improving blood pressure measurement accuracy. The present invention is defined by the appended claims.

According to a first aspect, this application provides a blood pressure measurement device. The blood pressure measurement device may include a body, a processor, an airbag, an air supply and exhaust apparatus, a driving apparatus, a barometric pressure sensor, and a flowmeter. The body includes a cavity, and function modules or components of the blood pressure measurement device may be disposed in the cavity. For example, the foregoing driving apparatus, the processor, the air supply and exhaust apparatus, and the barometric pressure sensor may be disposed in the cavity. The airbag is fastened to an end of the body, and the airbag has an air cavity. The air supply and exhaust apparatus includes an air inlet path and an air outlet path, and the air supply and exhaust apparatus is connected to the air cavity of the airbag through a first air path. The barometric pressure sensor may be connected to the air cavity of the airbag through a second air path, to detect an air pressure value in the air cavity. The flowmeter is configured to detect an air flow value of air flowing between the air supply and exhaust apparatus and the airbag, or is configured to detect an air flow value of air flowing between the barometric pressure sensor and the airbag. The blood pressure measurement device stores a correspondence between an air flow value and an air pressure compensation value, for example, stores the correspondence in the processor. The processor is electrically connected to the barometric pressure sensor, the flowmeter, and the driving apparatus. The processor may obtain an air pressure compensation value based on the air flow value detected by the flowmeter and the stored correspondence between an air flow value and an air pressure compensation value, to compensate, based on the obtained air pressure compensation value, the air pressure value detected by the barometric pressure sensor, and further, control the driving apparatus based on a compensated air pressure value. The driving apparatus is electrically connected to the air supply and exhaust apparatus, and is configured to drive, under control of the processor, the air supply and exhaust apparatus to perform inflating or deflating.

When the blood pressure measurement device is used for blood pressure measurement, when the air supply and exhaust apparatus inflates/deflates the airbag, because air flows between the air supply and exhaust apparatus, the airbag, and the barometric pressure sensor, the air flow value detected by the flowmeter may represent an inflating/deflating amount of the air supply and exhaust apparatus, so that the processor obtains the air pressure compensation value based on the air flow value detected by the flowmeter and the stored correspondence between an air flow value and an air pressure compensation value, and compensates, based on the obtained air pressure compensation value, the air pressure value detected by the barometric pressure sensor, to obtain an accurate air pressure value in the airbag. In this way, impact of the blood pressure measurement device on blood pressure measurement in an inflating/deflating process can be reduced, thereby improving blood pressure measurement accuracy.

In this application, to facilitate the flowmeter to detect an air flow value of air flowing between the air supply and exhaust apparatus and the airbag, the flowmeter may be disposed in the first air path, so as to detect an air flow value in the first air path. Alternatively, to facilitate the flowmeter to detect an air flow value of air flowing between the air supply and exhaust apparatus and the barometric pressure sensor, the flowmeter may be disposed in the second air path, so as to detect an air flow value in the second air path.

In this application, the air supply and exhaust apparatus and the pressure sensor may be directly connected to the airbag through a corresponding air path or indirectly connected to the airbag. For example, in a possible implementation of this application, the blood pressure measurement device may further include an air path cavity, and the air path cavity is disposed in the cavity of the body. In addition, the air supply and exhaust apparatus may be connected to the air path cavity through the first air path, the barometric pressure sensor may be connected to the air path cavity through the second air path, and the air path cavity is connected to the air cavity of the airbag through a third air path. In this way, the air paths that are of the air supply and exhaust apparatus and the barometric pressure sensor and that are used to be connected to the airbag may be first combined by using the air path cavity, and then connected to the airbag through one air path. In this case, only one through hole used to connect to the airbag needs to be disposed on a side wall of the body, so that a quantity of holes on the body can be reduced, and waterproof performance and structure stability of the blood pressure measurement device can be improved.

It should be noted that, in this application, when the blood pressure measurement device further includes the air path cavity, the flowmeter may alternatively be disposed in the third air path, to detect an air flow value in the third air path. In this way, the air flow value detected by the flowmeter may be an air flow value of air flowing between the air supply and exhaust apparatus and the airbag, or may be an air flow value of air flowing between the air supply and exhaust apparatus and the barometric pressure sensor.

In a possible implementation of this application, to connect the airbag to the body, a connection hole may be disposed at an end of the body. In addition, the airbag has an air nozzle, and the air nozzle protrudes from a side surface of the airbag in a direction towards the body. In this way, the air nozzle may be inserted in the connection hole, and the third air path is connected to the air nozzle, so as to implement connection between the airbag and the air path cavity. Similarly, in some other possible implementations, the air nozzle may alternatively be disposed at an end of the body, and a connection hole is disposed on the airbag. In this way, the airbag may alternatively be connected to the body through inserting connection between the air nozzle and the connection hole.

It should be noted that, in this application, the airbag may be detachably connected to the body. In this way, the airbag may be removed or replaced as required. In addition, in a possible implementation of this application, the blood pressure measurement device may further include a photoplethysmograph PPG module and an ECG detection module. The PPG module and the ECG detection module may be disposed on a bottom surface of the body. The airbag may alternatively be fastened to an end of the bottom surface of the body, so that the blood pressure measurement device integrates a plurality of measurement functions, and a structure of the blood pressure measurement device is compact.

In this application, the correspondence between an air flow value and an air pressure compensation value may be obtained in advance by analyzing pre-tested data, and may be pre-stored in the processor or a memory of the blood pressure measurement device before the blood pressure measurement device is delivered from a factory.

For example, in this application, the processor may obtain the correspondence between an air flow value and an air pressure compensation value in the following manner: first controlling the blood pressure measurement device to perform inflating when the airbag is removed, so that the air flow value detected by the flowmeter changes; obtaining a plurality of air pressure values detected by the barometric pressure sensor when the flowmeter detects a plurality of different air flow values, and using the obtained plurality of air pressure values as air pressure compensation values separately corresponding to the plurality of different air flow values; and finally, establishing the correspondence between an air flow value and an air pressure compensation value based on the plurality of different air flow values and the air pressure compensation values separately corresponding to the plurality of different air flow values.

For example, the processor may establish the correspondence between an air flow value and an air pressure compensation value in an interpolation method based on the plurality of different air flow values and the air pressure compensation values separately corresponding to the plurality of different air flow values.

It can be learned from the foregoing that the pre-established correspondence between an air flow value and an air pressure compensation value is stored. In this way, during blood pressure detection, the air pressure compensation value may be obtained based on the air flow value detected by the flowmeter and the stored correspondence between an air flow value and an air pressure compensation value, and the air pressure value detected by the barometric pressure sensor is compensated based on the obtained air pressure compensation value, to obtain an accurate air pressure value in the airbag.

A process in which the blood pressure measurement device performs blood pressure measurement may include: controlling the driving apparatus to drive the air supply and exhaust apparatus to inflate the airbag, reading an air pressure value detected by the barometric pressure sensor and an air flow value detected by the flowmeter, obtaining an air pressure compensation value based on the air flow value detected by the flowmeter and a stored correspondence between an air flow value and an air pressure compensation value, and compensating, based on the obtained air pressure compensation value, the air pressure value detected by the barometric pressure sensor. Specifically, the obtained air pressure compensation value may be subtracted from the air pressure value detected by the barometric pressure sensor, to obtain a compensated air pressure value. Then, an operating parameter (for example, a voltage, a current, or a frequency) of a driving circuit is controlled based on the compensated air pressure value to drive the air supply and exhaust apparatus to inflate the airbag. The foregoing procedure is performed at least once, for example, once, twice, three times, or four times, so that the compensated air pressure value meets a boost curve requirement for blood pressure measurement, thereby completing blood pressure measurement.

According to a second aspect, this application further provides another blood pressure measurement device. The blood pressure measurement device may include a body, a processor, an airbag, an air supply and exhaust apparatus, a driving apparatus, a first barometric pressure sensor, and a second barometric pressure sensor. The body includes a cavity, and function modules or components of the blood pressure measurement device may be disposed in the cavity. For example, the foregoing driving apparatus, the processor, the air supply and exhaust apparatus, the first barometric pressure sensor, and the second barometric pressure sensor may be disposed in the cavity. The airbag is fastened to an end of the body, and the airbag has an air cavity. The air supply and exhaust apparatus includes an air inlet path and an air outlet path, and the air supply and exhaust apparatus is connected to the air cavity of the airbag through a first air path. The first barometric pressure sensor may be connected to the air cavity of the airbag through a second air path, to detect an air pressure value in the air cavity. The second barometric pressure sensor is configured to detect an air pressure value in the cavity. The blood pressure measurement device stores a correspondence between a cavity air pressure value and an air pressure compensation value, for example, stores the correspondence in the processor or a memory. The processor is electrically connected to the first barometric pressure sensor, the second barometric pressure sensor, and the driving apparatus. The processor may obtain an air pressure compensation value based on an air pressure value detected by the second barometric pressure sensor and the stored correspondence between a cavity air pressure value and an air pressure compensation value, to compensate, based on the obtained air pressure compensation value, an air pressure value detected by the first barometric pressure sensor, and control the driving apparatus based on a compensated air pressure value. The driving apparatus is electrically connected to the air supply and exhaust apparatus, and is configured to drive, under control of the processor, the air supply and exhaust apparatus to perform inflating/deflating.

In the blood pressure measurement device, when the air supply and exhaust apparatus inflates/deflates the airbag, the second barometric pressure sensor detects an air pressure value in the cavity, so that the processor obtains an air pressure compensation value based on the air pressure value detected by the second barometric pressure sensor and the stored correspondence between a cavity air pressure value and an air pressure compensation value, and then compensates, based on the obtained air pressure compensation value, the air pressure value detected by the first barometric pressure sensor, to obtain an accurate air pressure value in the airbag. In this way, impact of the blood pressure measurement device on blood pressure measurement in an inflating/deflating process can be reduced, thereby improving blood pressure measurement accuracy.

In this application, because air fluctuation in the air cavity is caused by inflating/deflating of the air supply and exhaust apparatus, in an area closer to the air supply and exhaust apparatus, there is larger air pressure fluctuation. Therefore, the second barometric pressure sensor may be disposed close to the air supply and exhaust apparatus, and a closer distance between the second barometric pressure sensor and the air supply and exhaust apparatus indicates a more accurate measurement result.

In this application, the air supply and exhaust apparatus and the pressure sensor may be directly connected to the airbag through a corresponding air path or indirectly connected to the airbag. For example, in a possible implementation of this application, the blood pressure measurement device may further include an air path cavity, and the air path cavity is disposed in the cavity of the body. In addition, the air supply and exhaust apparatus may be connected to the air path cavity through the first air path, the first barometric pressure sensor may be connected to the air path cavity through the second air path, and the air path cavity is connected to the air cavity of the airbag through a third air path. In this way, the air paths that are of the air supply and exhaust apparatus and the first barometric pressure sensor and that are used to be connected to the airbag may be first combined by using the air path cavity, and then connected to the airbag through one air path. In this case, only one through hole used to connect to the airbag needs to be disposed on a side wall of the body, so that a quantity of holes on the body can be reduced, and waterproof performance and structure stability of the blood pressure measurement device can be improved.

For example, the correspondence between a cavity air pressure value and an air pressure compensation value may alternatively be obtained in advance through data analysis, and is pre-stored in the processor in the blood pressure measurement device. The correspondence between a cavity air pressure value and an air pressure compensation value may be pre-established before the blood pressure measurement device is delivered from a factory.

In a feasible implementation, the processor may obtain the correspondence between a cavity air pressure value and an air pressure compensation value in the following manner: first controlling the blood pressure measurement device to perform inflating when the airbag is removed, so that a first air pressure value detected by the second barometric pressure sensor changes; obtaining a plurality of second air pressure values detected by the first barometric pressure sensor when the second barometric pressure sensor detects a plurality of different first air pressure values, and using the obtained plurality of second air pressure values as air pressure compensation values separately corresponding to the plurality of different first air pressure values; and finally, establishing the correspondence between a cavity air pressure value and an air pressure compensation value based on the plurality of different first air pressure values and the air pressure compensation values separately corresponding to the plurality of different first air pressure values.

For example, the processor may establish the correspondence between a cavity air pressure value and an air pressure compensation value in an interpolation method based on the plurality of different first air pressure values and the air pressure compensation values separately corresponding to the plurality of different first air pressure values.

It can be learned from the foregoing that the pre-established correspondence between a cavity air pressure value and an air pressure compensation value is stored. In this way, during blood pressure detection, the air pressure compensation value may be obtained based on the air pressure value detected by the second barometric pressure sensor and the stored correspondence between a cavity air pressure value and an air pressure compensation value, and the air pressure value detected by the first barometric pressure sensor is compensated based on the obtained air pressure compensation value, to obtain an accurate air pressure value in the airbag. A process in which the blood pressure measurement device performs blood pressure measurement may include: controlling the driving apparatus to drive the air supply and exhaust apparatus to inflate the airbag, reading an air pressure value detected by the first barometric pressure sensor and an air pressure value detected by the second barometric pressure sensor, obtaining an air pressure compensation value based on the air pressure value detected by the second barometric pressure sensor and a stored correspondence between a cavity air pressure value and an air pressure compensation value, and compensating, based on the obtained air pressure compensation value, the air pressure value detected by the first barometric pressure sensor. Specifically, the obtained air pressure compensation value may be subtracted from the air pressure value detected by the first barometric pressure sensor, to obtain a compensated air pressure value. Then, an operating parameter (for example, a voltage, a current, or a frequency) of a driving circuit is controlled based on the compensated air pressure value to drive the air supply and exhaust apparatus to inflate the airbag. The foregoing procedure is performed at least once, for example, once, twice, three times, or four times, so that the compensated air pressure value meets a boost curve requirement for blood pressure measurement, thereby completing blood pressure measurement.

According to a third aspect, this application further provides another blood pressure measurement device. The blood pressure measurement device may include a body, a processor, an airbag, an air supply and exhaust apparatus, a driving apparatus, and a barometric pressure sensor. The body includes a cavity, and function modules or components of the blood pressure measurement device may be disposed in the cavity. For example, the foregoing driving apparatus, the processor, the air supply and exhaust apparatus, the first barometric pressure sensor, and the second barometric pressure sensor may be disposed in the cavity. The airbag is fastened to an end of the body, and the airbag has an air cavity. The air supply and exhaust apparatus includes an air inlet path and an air outlet path, and the air supply and exhaust apparatus is connected to the air cavity of the airbag through a first air path. The barometric pressure sensor may be connected to the air cavity of the airbag through a second air path, to detect an air pressure value in the air cavity. The blood pressure measurement device stores a correspondence between a driving state and an air pressure compensation value, for example, stores the correspondence in the processor. The processor is electrically connected to the barometric pressure sensor and the driving apparatus, and the processor may obtain a corresponding air pressure compensation value based on a driving state of the driving apparatus and the correspondence that is between a driving state and an air pressure compensation value and that is stored in the blood pressure measurement device, compensate, based on the obtained air pressure compensation value, an air pressure value detected by the barometric pressure sensor, and control the driving apparatus based on a compensated air pressure value. The driving apparatus is electrically connected to the air supply and exhaust apparatus, and is configured to drive, under control of the processor, the air supply and exhaust apparatus to perform inflating/deflating.

In the blood pressure measurement device, when the air supply and exhaust apparatus inflates/deflates the airbag, the processor may obtain the air pressure compensation value based on the driving state of the driving apparatus and the stored correspondence between a driving state and an air pressure compensation value, and then compensate, based on the obtained air pressure compensation value, the air pressure value detected by the barometric pressure sensor, to obtain an accurate air pressure value in the airbag. In this way, impact of the blood pressure measurement device on blood pressure measurement in an inflating/deflating process can be reduced, thereby improving blood pressure measurement accuracy.

It should be noted that the driving state may include at least one parameter related to a supply power, for example, a driving voltage, a driving current, and a duty cycle.

For example, an air path cavity may be disposed in the blood pressure measurement device, so that the air supply and exhaust apparatus is connected to the air path cavity through the first air path, the first barometric pressure sensor may be connected to the air path cavity through the second air path, and the air path cavity is connected to the airbag through a third air path, so as to implement a single-air nozzle connection between the body and the airbag.

In this application, the correspondence between a driving state and an air pressure compensation value may be obtained in advance by analyzing pre-tested data, and is pre-stored in the processor of the blood pressure measurement device.

For example, in this application, the processor may obtain the correspondence between a driving state and an air pressure compensation value in the following manner: controlling the blood pressure measurement device to perform inflating in a plurality of different driving states when the airbag is removed; then obtaining a plurality of air pressure values detected by the barometric pressure sensor when the blood pressure measurement device performs driving in the plurality of different driving states, and using the obtained plurality of air pressure values as air pressure compensation values separately corresponding to the plurality of different driving states; and finally, establishing the correspondence between a driving state and an air pressure compensation value based on the plurality of different driving states and the air pressure compensation values separately corresponding to the plurality of different driving states.

For example, the processor may establish the correspondence between a driving state and an air pressure compensation value in an interpolation method based on the plurality of different driving states and the air pressure compensation values separately corresponding to the plurality of different driving states.

It can be learned from the foregoing that the pre-established correspondence between a driving state and an air pressure compensation value is stored. In this way, during blood pressure detection, the air pressure compensation value may be obtained based on the driving state and the stored correspondence between a driving state and an air pressure compensation value, and the air pressure value detected by the barometric pressure sensor is compensated based on the obtained air pressure compensation value, to obtain an accurate air pressure value in the airbag.

A process in which the blood pressure measurement device performs blood pressure measurement may include: controlling the driving apparatus to drive the air supply and exhaust apparatus to inflate the airbag, reading an air pressure value detected by the barometric pressure sensor, obtaining an air pressure compensation value based on a driving state of the driving apparatus and a stored correspondence between a driving state and an air pressure compensation value, and compensating, based on the obtained air pressure compensation value, the air pressure value detected by the barometric pressure sensor. Specifically, the obtained air pressure compensation value may be subtracted from the air pressure value detected by the barometric pressure sensor, to obtain a compensated air pressure value. Then, an operating parameter (for example, a voltage, a current, or a frequency) of a driving circuit is controlled based on the compensated air pressure value to drive the air supply and exhaust apparatus to inflate the airbag. The foregoing procedure is performed at least once, for example, once, twice, three times, or four times, so that the compensated air pressure value meets a boost curve requirement for blood pressure measurement, thereby completing blood pressure measurement.

According to a fourth aspect, this application further provides an electronic device, and the electronic device may include the blood pressure measurement device provided in any one of the implementations in the first aspect to the third aspect.

For technical effects that can be achieved in the fourth aspect, refer to descriptions of technical effects that can be achieved in any one of the possible designs in the first aspect to the third aspect. Details are not described herein again.

### BRIEF DESCRIPTION OF DRAWINGS

FIG. 1 is a schematic diagram of a structure of a blood pressure measurement device according to an embodiment of this application;
FIG. 2 is a schematic diagram of a framework structure of an existing blood pressure measurement device according to an embodiment of this application;
FIG. 3 is a schematic diagram of a framework structure of a blood pressure measurement device according to an embodiment of this application;
FIG. 4 is a schematic diagram of a framework structure of a blood pressure measurement device according to another embodiment of this application;
FIG. 5 is a schematic diagram of a framework structure of a blood pressure measurement device according to another embodiment of this application;
FIG. 6 is a schematic diagram of a framework structure of a blood pressure measurement device according to another embodiment of this application;
FIG. 7 is a schematic diagram of a framework structure of a blood pressure measurement device according to another embodiment of this application;
FIG. 8 is a schematic flowchart of a blood pressure measurement method according to an embodiment of this application;
FIG. 9 is a schematic flowchart of establishing a correspondence between an air flow value and an air pressure compensation value according to an embodiment of this application;
FIG. 10 is a schematic diagram of a curve of a correspondence between an air flow value and an air pressure compensation value according to an embodiment of this application;
FIG. 11 is a schematic diagram of a framework structure of a blood pressure measurement device according to another embodiment of this application;
FIG. 12 is a schematic diagram of a framework structure of a blood pressure measurement device according to another embodiment of this application;
FIG. 13 is a schematic flowchart of a blood pressure measurement method according to another embodiment of this application;
FIG. 14 is a schematic flowchart of establishing a correspondence between a cavity air pressure value and an air pressure compensation value according to an embodiment of this application;
FIG. 15 is a schematic diagram of a curve of a correspondence between a cavity air pressure value and an air pressure compensation value according to an embodiment of this application;
FIG. 16 is a schematic diagram of a framework structure of a blood pressure measurement device according to another embodiment of this application;
FIG. 17 is a schematic diagram of a framework structure of a blood pressure measurement device according to another embodiment of this application;
FIG. 18 is a schematic flowchart of a blood pressure measurement method according to another embodiment of this application;
FIG. 19 is a schematic flowchart of establishing a correspondence between a driving state and an air pressure compensation value according to an embodiment of this application; and
FIG. 20 is a schematic diagram of a curve of a correspondence between a driving power and an air pressure compensation value according to an embodiment of this application.

Reference numerals:
1: body; 101: cavity;
102: PPG module; 103: ECG detection module;
2: airbag; 3: wrist strap;
4: air supply and exhaust apparatus; 401: air inlet path;
402: air outlet path; 5: barometric pressure sensor;
5a: first barometric pressure sensor; 5b: second barometric pressure sensor;
6: driving apparatus; 7: processor;
81: first air path; 82: second air path;
83: third air path; 9: flowmeter; and
10: air path cavity.

### DESCRIPTION OF EMBODIMENTS

To make the objectives, technical solutions, and advantages of this application clearer, the following further describes this application in detail with reference to the accompanying drawings.

To facilitate understanding of the blood pressure measurement device provided in embodiments of this application, the following first describes an application scenario of the blood pressure measurement device. The blood pressure measurement device may be, but is not limited to, a device with a large volume used for blood pressure measurement, for example, a medical device or a household device, or may be a portable electronic device with a blood pressure measurement function, for example, a smart watch or a smart band. The smart watch is used as an example. The smart watch may be worn on a wrist of a user, to detect a physical sign, for example, blood pressure of the user at any time, so as to predict a physical state, thereby effectively avoiding a dangerous secondary disease, for example, a stroke caused by hypertension.

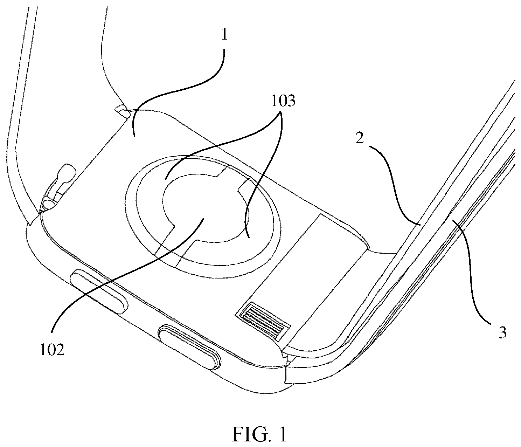

FIG. 1 is a schematic diagram of a structure of a smart watch with a blood pressure measurement function according to an embodiment of this application. A blood pressure measurement device with a blood pressure detection function may usually include a body 1 and an airbag 2, and the airbag 2 may be fastened to an end of the body 1. For example, the airbag 2 may be fastened to an end face of a bottom surface of the body 1. In this application, the bottom surface of the body 1 is a surface on which the body 1 is directly in contact with a wrist when the smart watch is worn on the wrist. In addition, the blood pressure measurement device may further include a wrist strap 3. As shown in FIG. 1, the airbag 2 may be located on a side that is of the wrist strap 3 and that faces a user. In this way, when the wrist strap 3 is wrapped around the wrist of the user, the airbag 2 may be pressed to the wrist, and the airbag 2 is attached to the wrist, thereby facilitating blood pressure measurement of the user. It may be understood that the airbag 2 and the wrist strap 3 may be fastened in a manner of but not limited to clamping, bonding, or riveting, to reduce friction generated when the airbag 2 and the wrist strap 3 move mutually, thereby reducing a risk of airbag wear and improving a service life of the blood pressure measurement device.

In addition to the foregoing structure, the smart watch with the blood pressure measurement function may be generally provided with a photoplethysmograph (photoplethysmograph, PPG) module. The PPG module 102 may alternatively be disposed on the bottom surface of the body 1. In addition, the PPG module 102 may be disposed in a middle area (refer to a middle circular area of the bottom surface of the body 1 shown in FIG. 1) of the bottom surface of the body 1, to improve detection accuracy of the PPG module 102. Because the PPG module 102 may continuously measure a heart rate value of a human body, by disposing both the airbag 2 and the PPG module 102 in the smart watch, a function of performing a single time of blood pressure measurement of the airbag 2 may be integrated with a continuous heart rate measurement function of the PPG module 102, and a problem of continuous blood pressure measurement is resolved by using an accurate algorithm operation.

Still refer to FIG. 1. An electrocardiogram (electrocardiogram, ECG) detection module 103 may further be disposed in the smart watch in this embodiment of this application. The ECG detection module 103 may alternatively be disposed on the bottom surface of the body 1. In addition, the ECG detection module 103 may be disposed in a middle area of the body 1. For example, the ECG detection module 103 may be disposed at a periphery side of the PPG module 102 (refer to two arc areas in the middle of the bottom surface of the body 1 shown in FIG. 1). In this way, an electrocardiogram detection function of the smart watch is implemented.

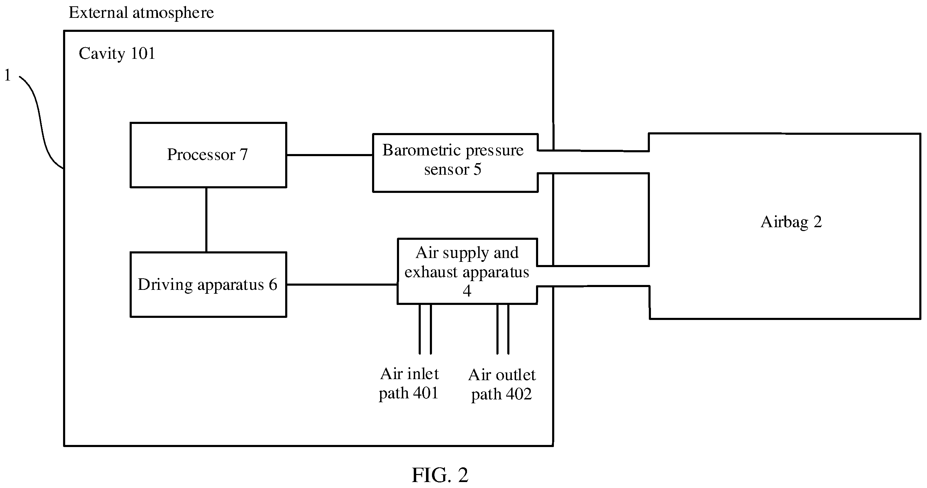

FIG. 2 is a schematic diagram of a framework structure of a conventional blood pressure measurement device. The body 1 has a cavity 101, and main function modules and components (such as circuit components like a processor and a sensor) of the blood pressure measurement device may be disposed in the cavity 101 of the body 1, for example, an air supply and exhaust apparatus 4, a barometric pressure sensor 5, a driving apparatus 6, and a processor 7. An end of the airbag 2 may be connected to the air supply and exhaust apparatus 4 and the barometric pressure sensor 5 through an air nozzle, and the airbag 2 may be wrapped around the wrist of the user. When the blood pressure measurement device is used to perform blood pressure measurement, the processor 7 controls the driving apparatus 6 to drive the air supply and exhaust apparatus 4 to inflate/deflate the airbag 2, and the barometric pressure sensor 5 may detect a change of air pressure in the airbag 2 in the foregoing inflating/deflating process. In this way, a blood pressure value of the user may be obtained by performing an operation on the detected air pressure value by using an algorithm.

It may be understood from the foregoing description of the process in which the blood pressure measurement device performs blood pressure measurement that, because the air supply and exhaust apparatus 4 is disposed in the cavity 101 of the body 1, in a process in which the air supply and exhaust apparatus 4 inflates/deflates the airbag 2, air pressure in the cavity 101 of the body 1 fluctuates. The air pressure fluctuation causes a difference between an air pressure value detected by the barometric pressure sensor 5 and an actual air pressure value in the airbag 2. In addition, the air pressure value detected by the barometric pressure sensor 5 is usually greater than an actual air pressure value inside the airbag 2.

Based on this, an embodiment of this application provides a blood pressure measurement device, to reduce impact of air pressure in the cavity 101 of the body 1 of the blood pressure measurement device on blood pressure measurement of the blood pressure measurement device, thereby improving blood pressure measurement accuracy. For ease of understanding, in the following embodiments of this application, a specific structure of the blood pressure measurement device is described in detail by using a smart watch as an example.

Terms used in the following embodiments are merely intended to describe specific embodiments, but are not intended to limit this application. Terms "one", "a", "the foregoing", "the", and "the one" of singular forms used in this specification and the appended claims of this application are also intended to include plural forms like "one or more", unless otherwise specified in the context clearly. It should be further understood that in the following embodiments of this application, "at least one" and "one or more" refer to one, two, or more. The term "and/or" is used for describing an association relationship between associated objects, and indicates that three relationships may exist. For example, A and/or B may represent: Only A exists, both A and B exist, and only B exists, where A and B may be singular or plural. The character "/" generally indicates an "or" relationship between the associated objects.

Reference to "one embodiment" or "some embodiments" described in this specification means that a specific characteristic, structure or feature described in combination with this embodiment is included in one or more embodiments of this application. Therefore, statements such as "in an embodiment", "in some embodiments", "in some other embodiments", and "in other embodiments" that appear at different places in this specification do not necessarily mean referring to a same embodiment. Instead, the statements mean "one or more but not all of embodiments", unless otherwise specifically emphasized in another manner. The terms "include", "comprise", "have", and their variants all mean "include but are not limited to", unless otherwise specifically emphasized in another manner.

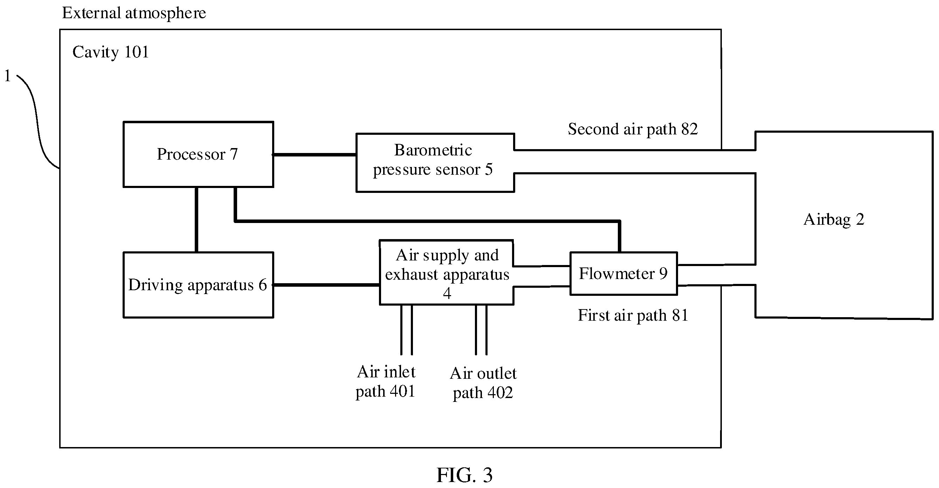

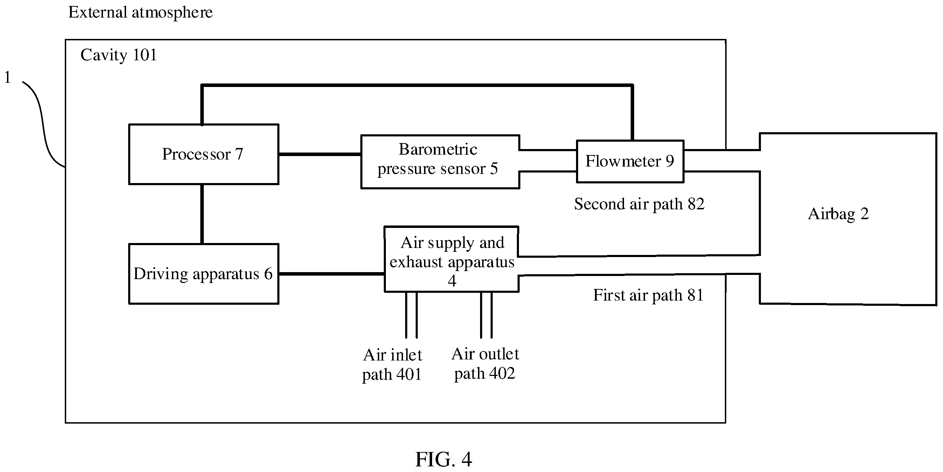

Refer to FIG. 3 and FIG. 4. FIG. 3 is a schematic diagram of a framework structure of a blood pressure measurement device according to an embodiment of this application, and FIG. 4 is a schematic diagram of a framework structure of a blood pressure measurement device according to another embodiment of this application. In this embodiment of this application, the blood pressure measurement device may include a body 1 and an airbag 2. The body 1 has a plurality of side walls, and the plurality of side walls are connected to enclose a cavity 101 of the body 1. Main function modules and components of the blood pressure measurement device may be disposed in the cavity 101 of the body 1. The airbag 2 may be fastened to a side wall of the body 1, and the airbag 2 has an air cavity.

Still refer to FIG. 3 and FIG. 4. In this embodiment of this application, the blood pressure measurement device may further include an air supply and exhaust apparatus 4 and a barometric pressure sensor 5. Both the air supply and exhaust apparatus 4 and the barometric pressure sensor 5 are disposed in the cavity 101 of the body 1. The air supply and exhaust apparatus 4 includes an air inlet path 401 and an air outlet path 402, where both the air inlet path 401 and the air outlet path 402 are connected to the cavity 101 of the body 1. In addition, the air supply and exhaust apparatus 4 is further connected to the air cavity of the airbag 2 through a first air path 81. In this way, air in the cavity 101 of the body 1 may enter the air supply and exhaust apparatus 4 through the air inlet path 401, and enter the airbag 2 through the first air path 81. In this way, the air supply and exhaust apparatus 4 inflates the airbag 2. On the contrary, when air in the airbag 2 needs to be exhausted, the air supply and exhaust apparatus 4 extracts the air in the airbag 2 through the first air path 81, and exhausts the air to the cavity 101 of the body 1 through the air outlet path 402.

In addition, because the air inlet path 401 and the air outlet path 402 of the air supply and exhaust apparatus 4 do not work at the same time, in a possible embodiment of this application, the air inlet path 401 and the air outlet path 402 may be combined, that is, only one air path is disposed in the air supply and exhaust apparatus 4, the air supply and exhaust apparatus 4 may inflate the airbag 2 through the air path, and the air in the airbag 2 may be extracted through the air path. Therefore, a structure of the blood pressure measurement device can be simplified.

In this application, a specific structure of the air supply and exhaust apparatus 4 is not limited. For example, the air supply and exhaust apparatus 4 may be an air pump, and a volume of the air pump may be set based on a requirement of the airbag 2 of the blood pressure measurement apparatus for air supplying and exhausting and a size of the cavity 101 of the body 1. In addition, considering that a volume of a body 1 of an existing smart watch with a blood pressure measurement function is small, space of a cavity 101 of the smart watch is also small. Therefore, a volume of an air pump disposed in the smart watch is also small. In some possible embodiments of this application, the air pump may be fastened to a mechanical part (not shown in the figure), and then the air pump is installed in the body 1 by fastening the mechanical part to the body 1. A material of the mechanical part may be, but is not limited to, a metal or a nonmetal with high strength, so that the mechanical part can reliably support the air pump, thereby improving structure reliability of the air pump. In this application, a manner of fastening the air pump and the mechanical part is not limited. For example, the air pump and the mechanical part may be fastened through glue dispensing, threaded connection, or the like. In addition, in some embodiments of this application, glue dispensing may be further performed around the air pump, to perform glue sealing on the air pump, so as to improve structure stability of the air pump.

Still refer to FIG. 3 and FIG. 4. The blood pressure measurement device may further include a driving apparatus 6 and a processor 7, and the processor 7 is electrically connected to the driving apparatus 6 and the barometric pressure sensor 5 separately. In this application, locations of the driving apparatus 6 and the processor 7 are not limited. For example, the driving apparatus 6 and the processor 7 may be disposed in the cavity 101 of the body 1. The processor 7 is configured to control the barometric pressure sensor 5 and the driving apparatus 6. The driving apparatus 6 is configured to provide a driving force for an inflating/deflating process of the air supply and exhaust apparatus 4 under control of the processor 7. In this application, the driving apparatus 6 is not specifically limited either. For example, the driving apparatus 6 may be a motor. The barometric pressure sensor 5 is configured to perform air pressure detection under control of the processor 7. In this application, the barometric pressure sensor 5 is not specifically limited. In this application, because the air supply and exhaust apparatus 4 is disposed in the cavity 101 of the body 1, in a process in which the air supply and exhaust apparatus 4 inflates/deflates the airbag 2, air pressure in the cavity 101 of the body 1 fluctuates. The air pressure fluctuation is related to an inflating/deflating amount of the air supply and exhaust apparatus 4. If a change of an air pressure value of the barometric pressure sensor 5 that is caused by the inflating/deflating amount can be obtained, error compensation may be performed on the air pressure value detected by the barometric pressure sensor 5, to reduce impact of air pressure in the cavity 101 of the body 1 of the blood pressure measurement device on blood pressure measurement of the barometric pressure sensor, and improve blood pressure measurement accuracy.

Still refer to FIG. 3 and FIG. 4. Therefore, the blood pressure measurement device may further include a flowmeter 9. The flowmeter 9 is configured to detect an air flow value of air flowing between the air supply and exhaust apparatus 4 and the airbag 2, or is configured to detect an air flow value of air flowing between the barometric pressure sensor 5 and the airbag 2. The blood pressure measurement device stores a correspondence between an air flow value and an air pressure compensation value. For example, the correspondence may be stored in the processor 7 or a memory. The processor 7 may obtain a corresponding air pressure compensation value based on the air flow value detected by the flowmeter 9 and the stored correspondence between an air flow value and an air pressure compensation value, to compensate, based on the obtained air pressure compensation value, the air pressure value detected by the barometric pressure sensor 5. In this way, the driving apparatus 6 may be controlled based on a compensated air pressure value. The driving apparatus 6 drives, under control of the processor 7, the air supply and exhaust apparatus 4 to perform inflating or deflating, that is, the driving apparatus 6 may be configured to provide a driving force for the inflating/deflating process of the air supply and exhaust apparatus 4.

It can be learned that, in this application, when the air supply and exhaust apparatus 4 inflates/deflates the airbag 2, because air flows between the air supply and exhaust apparatus 4, the airbag 2, and the barometric pressure sensor 5, the air flow value detected by the flowmeter 9 may represent the inflating/deflating amount of the air supply and exhaust apparatus 4, so that the processor 7 obtains the air pressure compensation value based on the air flow value detected by the flowmeter 9 and the stored correspondence between an air flow value and an air pressure compensation value, and compensates, based on the obtained air pressure compensation value, the air pressure value detected by the barometric pressure sensor 5, to obtain an accurate air pressure value in the airbag 2. In this way, impact of the blood pressure measurement device on blood pressure measurement in an inflating/deflating process can be reduced, thereby improving blood pressure measurement accuracy.

Refer to FIG. 3. In this application, to facilitate the flowmeter 9 to detect an air flow value of air flowing between the air supply and exhaust apparatus 4 and the airbag 2, for example, the flowmeter 9 may be disposed in the first air path 81, so as to detect an air flow value in the first air path 81. Refer to FIG. 4. Alternatively, in this application, to facilitate the flowmeter 9 to detect an air flow value of air flowing between the air supply and exhaust apparatus 4 and the barometric pressure sensor 5, for example, the flowmeter 9 may be disposed in a second air path 82, so as to detect an air flow value in the second air path 82.

In this application, because air fluctuation in the air cavity is caused by inflating/deflating of the air supply and exhaust apparatus 4, in an area closer to the air supply and exhaust apparatus 4, there is larger air pressure fluctuation. Therefore, the flowmeter 9 may be disposed in the first air path 81 that is closer to the air supply and exhaust apparatus 4 to improve measurement accuracy compared with disposing the flowmeter 9 in the second air path 82.

In embodiments of this application, a specific setting form of each air path is not limited. The air path may be set in a straight line, or may be set in a curved device. Specifically, adaptive adjustment may be performed based on a component in internal space of the blood pressure measurement device. It may be understood that, to make the figures clear, in schematic diagrams of this application, each air path is represented as a straight line.

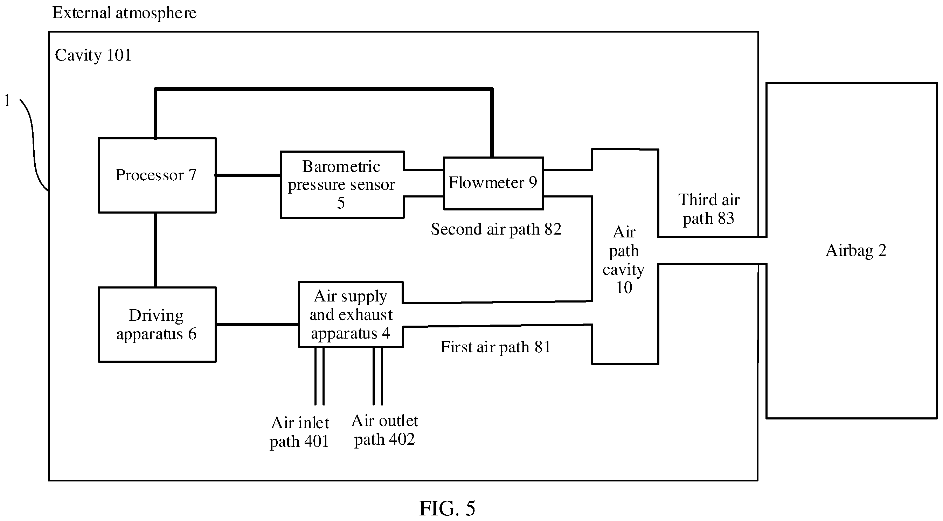

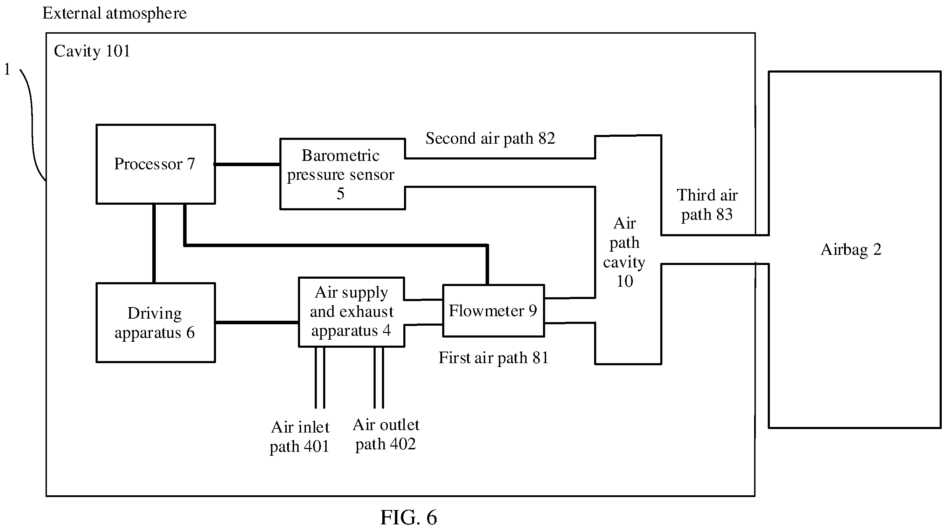

Refer to FIG. 5 and FIG. 6. FIG. 5 is a schematic diagram of a framework structure of a blood pressure measurement device according to another possible embodiment of this application. FIG. 6 is a schematic diagram of a framework structure of a blood pressure measurement device according to another possible embodiment of this application. A main difference between the blood pressure measurement devices in the embodiments shown in FIG. 5 and FIG. 6 and the embodiments shown in FIG. 3 and FIG. 4 lies in that in the embodiments shown in FIG. 5 and FIG. 6, the blood pressure measurement device further includes an air path cavity 10, and the air path cavity 10 is disposed in a cavity 101 of a body 1. In addition, the air path cavity 10 may be fastened, but not limited to, to a side that is of a side wall of the body 1 and that faces the cavity 101 through bonding or thread connection, to improve structure stability of the air path cavity 10.

Based on the foregoing change of the structure of the blood pressure measurement device, in this embodiment of this application, a connection manner between a barometric pressure sensor 5 and an air supply and exhaust apparatus 4 and a connection manner between the barometric pressure sensor 5 and an airbag 2 are also adaptively changed. Still refer to FIG. 5 and FIG. 6. During specific implementation, an air supply and exhaust apparatus 4 is connected to the air path cavity 10 through a first air path 81, and the barometric pressure sensor 5 is connected to the air path cavity 10 through a second air path 82. In this way, the first air path 81 and the second air path 82 may be connected through the air path cavity 10. In addition, the air path cavity 10 may be connected to an air cavity of an airbag 2 through a third air path 83. Other structure of the blood pressure measurement devices in the embodiments shown in FIG. 5 and FIG. 6 may be set with reference to any one of the foregoing embodiments, and details are not described herein again.

By using the blood pressure measurement device provided in this embodiment of this application, by adding the air path cavity 10, an air inlet path 401 and an air outlet path 402 of the air supply and exhaust apparatus 4 may be connected to the air cavity of the airbag 2 through the air path cavity 10. When the air supply and exhaust apparatus 4 works, the air supply and exhaust apparatus 4 may inhale air from the cavity 101 of the body 1 to the air path cavity 10, and then the air enters the air cavity of the airbag 2. In addition, the air supply and exhaust apparatus 4 may further exhaust air in the airbag 2 by exhausting the air in the air path cavity 10 through the air outlet path 402.

In a possible implementation of this application, to connect the airbag to the body, a connection hole may be disposed at an end of the body. In addition, the airbag has an air nozzle, and the air nozzle protrudes from a side surface of the airbag in a direction towards the body. In this way, the airbag may be connected to the connection hole by inserting the air nozzle in the connection hole, and the third air path is connected to the air nozzle, so as to implement connection between the airbag and the air path cavity. Similarly, in some other possible implementations, the air nozzle may alternatively be disposed at an end of the body, and a connection hole is disposed on the airbag. In this way, the airbag may alternatively be connected to the body through inserting connection between the air nozzle and the connection hole.

It should be noted that, in this application, the airbag may be detachably connected to the body. In this way, the airbag may be removed or replaced as required. In addition, in a possible implementation of this application, the blood pressure measurement device may further include a photoplethysmograph PPG module and an ECG detection module. The PPG module and the ECG detection module may be disposed on the bottom surface of the body. The airbag may alternatively be fastened to an end of the bottom surface of the body, so that the blood pressure measurement device integrates a plurality of measurement functions, and a structure of the blood pressure measurement device is compact.

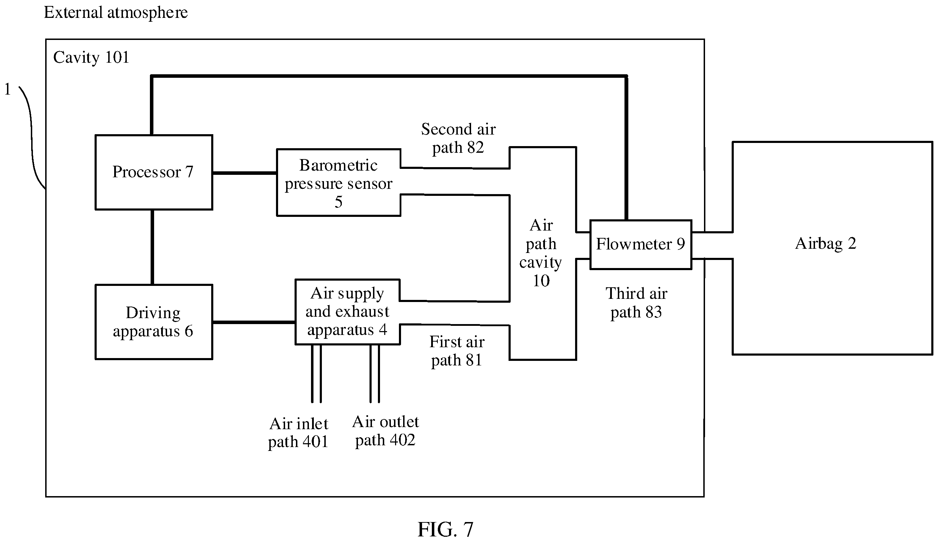

FIG. 7 is a schematic diagram of a framework structure of a blood pressure measurement device according to another possible embodiment of this application. When the blood pressure measurement device further includes an air path cavity 10, a flowmeter 9 may alternatively be disposed in a third air path 83, and is configured to detect an air flow value in the third air path 83. In this way, the air flow value detected by the flowmeter 9 may be an air flow value of air flowing between an air supply and exhaust apparatus 4 and an airbag 2, or may be an air flow value of air flowing between the air supply and exhaust apparatus 4 and a barometric pressure sensor 5. It should be noted that, compared with the blood pressure measurement devices in the embodiments shown in FIG. 5 and FIG. 6, the blood pressure measurement device in the embodiment shown in FIG. 7 has a same working principle but a different location of the flowmeter 9. Another structure of the blood pressure measurement device in the embodiment shown in FIG. 7 may be set with reference to any one of the foregoing embodiments.

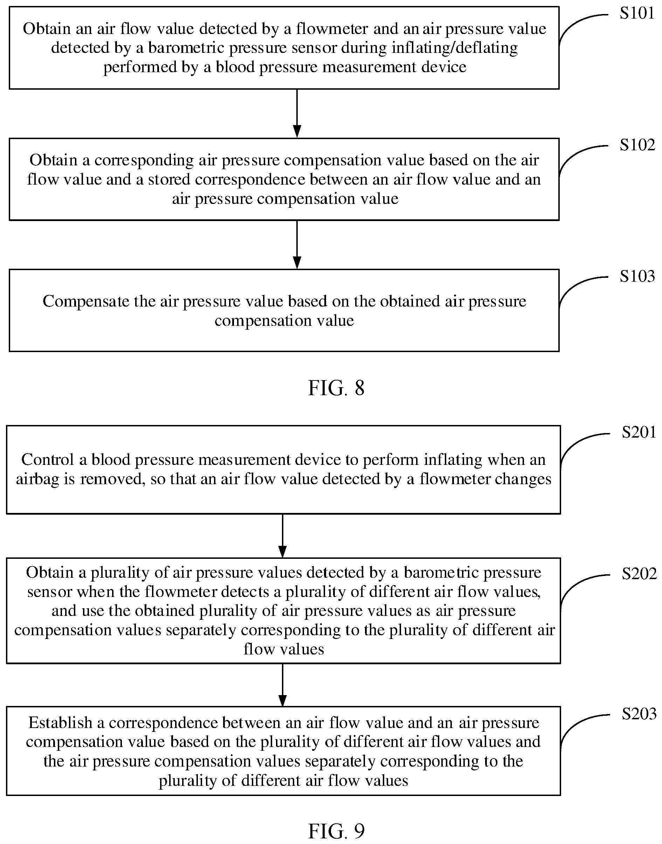

FIG. 8 is a blood pressure measurement method of a blood pressure measurement device according to an embodiment of this application. The blood pressure measurement device mainly includes a body, an airbag, a barometric pressure sensor, and a flowmeter. The body includes a cavity, the barometric pressure sensor is located in the cavity, the airbag has an air cavity, and the air cavity is connected to the cavity. The flowmeter is configured to detect an air flow value during inflating/deflating performed by the blood pressure measurement device. The barometric pressure sensor is configured to detect an air pressure value in the air cavity. For specific implementation of the blood pressure measurement device, refer to the blood pressure measurement device in the embodiments shown in FIG. 3 to FIG. 7. Details are not described herein again. As shown in FIG. 8, the blood pressure measurement method of the blood pressure measurement device may include the following steps.

S101: Obtain an air flow value detected by the flowmeter and an air pressure value detected by the barometric pressure sensor during inflating/deflating performed by the blood pressure measurement device.

S102: Obtain a corresponding air pressure compensation value based on the air flow value and a stored correspondence between an air flow value and an air pressure compensation value.

S103: Compensate the air pressure value based on the obtained air pressure compensation value.

It can be learned that, during blood pressure measurement, the air pressure compensation value may be obtained based on the air flow value detected by the flowmeter and the stored correspondence between an air flow value and an air pressure compensation value, and the air pressure value detected by the barometric pressure sensor is compensated based on the obtained air pressure compensation value, to obtain an accurate air pressure value in the airbag. In this way, impact of air pressure in the cavity on blood pressure measurement can be reduced, thereby improving blood pressure measurement accuracy.

In this application, the correspondence between an air flow value and an air pressure compensation value may be obtained in advance by analyzing pre-tested data, and may be pre-stored in the processor or a memory of the blood pressure measurement device before the blood pressure measurement device is delivered from a factory.

For example, as shown in FIG. 9, the processor may obtain the correspondence between an air flow value and an air pressure compensation value in the following manner: Step S201: Control the blood pressure measurement device to perform inflating when the airbag is removed, so that the air flow value detected by the flowmeter changes.

The airbag in the blood pressure measurement device is removed, so that the cavity of the body can be connected to the outside atmosphere, thereby ensuring that the air pressure value detected by the barometric pressure sensor is not affected by the air pressure in the air cavity of the airbag, and is mainly generated by air flow fluctuation in the cavity.

During specific implementation, the driving apparatus may be controlled to change the air flow value detected by the flowmeter from a minimum value to a maximum value.

Step S202: Obtain a plurality of air pressure values detected by the barometric pressure sensor when the flowmeter detects a plurality of different air flow values, and use the obtained plurality of air pressure values as air pressure compensation values separately corresponding to the plurality of different air flow values.

The plurality of different air flow values may be selected from zero to the maximum air flow value. For example, if the maximum value of the air flow value detected by the flowmeter is 100 ml/min, the plurality of air flow values may be selected from 0 to 100 ml/min. For example, the selected air flow values may be evenly distributed, for example, 0 ml/min, 10 ml/min, 20 ml/min, 30 ml/min, 40 ml/min, 50 ml/min, 60 ml/min, 70 ml/min, 80 ml/min, 90 ml/min and 100 ml/min. It may be understood when a quantity of selected air flow values is larger, a subsequently obtained correspondence between an air flow value and an air pressure compensation value is more accurate.

During specific implementation, when the air flow value detected by the flowmeter is q1 (for example, 0 ml/min), an air pressure value Δp1 detected by the barometric pressure sensor may be obtained, and Δp1 is used as an air pressure compensation value corresponding to q1. For example, Δp1 may be recorded and stored in a memory for subsequent use. Then, air flow is increased, and when the air flow value detected by the flowmeter is q2 (for example, 10 ml/min), an air pressure value Δp2 detected by the barometric pressure sensor is obtained, and Δp2 is used as an air pressure compensation value corresponding to q2. For example, Δp2 may be recorded and stored in the memory for subsequent use. Then, air flow is increased, and when the air flow value detected by the flowmeter is q3 (for example, 20 ml/min), an air pressure value Δp3 detected by the barometric pressure sensor is obtained, and Δp3 is used as an air pressure compensation value corresponding to q3. For example, Δp3 may be recorded and stored in the memory for subsequent use. By analogy, N different air flow values are selected from 0 to the maximum air flow value: q1, q2, q3, q4, ..., and qN, and corresponding air pressure compensation values Δp1, Δp2, Δp3, Δp4, ..., and ΔpN are obtained. qN may be the maximum air flow value.

Step S203: Establish the correspondence between an air flow value and an air pressure compensation value based on the plurality of different air flow values and the air pressure compensation values separately corresponding to the plurality of different air flow values.

For example, the processor may establish the correspondence between an air flow value and an air pressure compensation value in an interpolation method based on the plurality of different air flow values and the air pressure compensation values separately corresponding to the plurality of different air flow values.

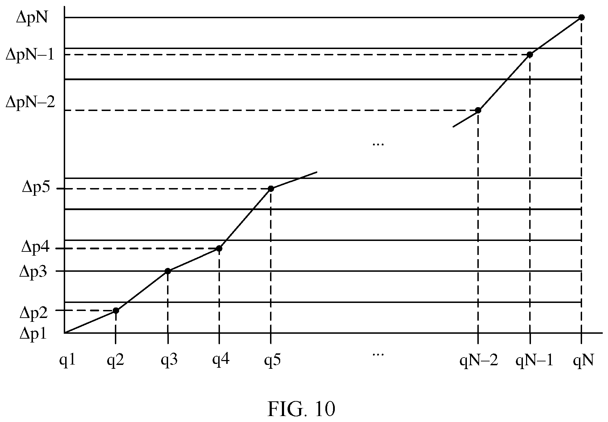

Refer to FIG. 10, for an air flow value q(x) between q1 and q2, q2 and q3, ..., and qN-1 and qN, for example, q(x) is between qi and qj, an air pressure compensation value Δp(x) corresponding to q(x) may be determined according to a formula Δp(x)=Δpi+[q(x)-qi](Δpj-Δpi)/(qj-qi), where i may be any number from 1 to N-1, and j=1+1.

During specific implementation, the air pressure compensation value Δp(x) corresponding to the air flow value q(x) located between 0 and q1, q1 and q2, q2 and q3, ..., and qN-1 and qN may alternatively be confirmed in another manner. For example, if q(x) is located between qi and qj, the air pressure compensation value Δp(x) corresponding to q(x) is equal to (Δpj-Δpi)/2. i may be any number from 1 to N-1, and j=1+1.

It can be learned from the foregoing that the pre-established correspondence between an air flow value and an air pressure compensation value is stored. In this way, during blood pressure detection, the air pressure compensation value may be obtained based on the air flow value detected by the flowmeter and the stored correspondence between an air flow value and an air pressure compensation value, and the air pressure value detected by the barometric pressure sensor is compensated based on the obtained air pressure compensation value, to obtain an accurate air pressure value in the airbag. A process in which the blood pressure measurement device performs blood pressure measurement may include: controlling the driving apparatus to drive the air supply and exhaust apparatus to inflate the airbag, reading an air pressure value detected by the barometric pressure sensor and an air flow value detected by the flowmeter, obtaining an air pressure compensation value based on the air flow value detected by the flowmeter and a stored correspondence between an air flow value and an air pressure compensation value, and compensating, based on the obtained air pressure compensation value, the air pressure value detected by the barometric pressure sensor. Specifically, the obtained air pressure compensation value may be subtracted from the air pressure value detected by the barometric pressure sensor, to obtain a compensated air pressure value. Then, an operating parameter (for example, a voltage, a current, or a frequency) of a driving circuit is controlled based on the compensated air pressure value to drive the air supply and exhaust apparatus to inflate the airbag. The foregoing procedure is performed at least once, for example, once, twice, three times, or four times, so that the compensated air pressure value meets a boost curve requirement for blood pressure measurement, thereby completing blood pressure measurement.

It should be noted that, this application is not limited to using the flowmeter to directly detect the air flow during inflating performed by the blood pressure measurement device, and another sensor that can indirectly reflect an air flow may be used to replace the flowmeter, for example, a barometric pressure sensor is used for implementation. For details, refer to the following embodiments.

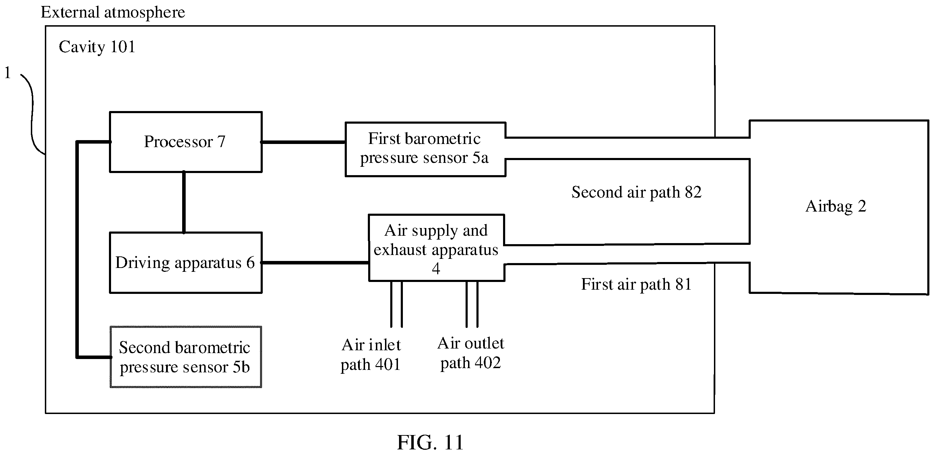

FIG. 11 is a schematic diagram of a structure of a blood pressure measurement device according to another embodiment of this application. A structure of the blood pressure measurement device in this embodiment is different from that in any one of the foregoing embodiments. A main difference lies in that the blood pressure measurement device does not include a flowmeter, but includes two barometric pressure sensors, namely, a first barometric pressure sensor 5a and a second barometric pressure sensor 5b. In the blood pressure measurement device, both the first barometric pressure sensor 5a and the second barometric pressure sensor 5b are disposed in a cavity 101. The first barometric pressure sensor 5a may be connected to an air cavity of an airbag 2 through a second air path 82, to detect an air pressure value in an air cavity of the airbag. The second barometric pressure sensor 5b is configured to detect an air pressure value in the cavity 101 of a body 1. The blood pressure measurement device stores a correspondence between a cavity air pressure value and an air pressure compensation value, for example, may store the correspondence in a processor or a memory. A processor 7 is electrically connected to the first barometric pressure sensor 5a, the second barometric pressure sensor 5b, and a driving apparatus 6, and is configured to: obtain a corresponding air pressure compensation value based on the air pressure value detected by the second barometric pressure sensor 5b and a correspondence that is stored in the blood pressure measurement device and that is between a cavity air pressure value and an air pressure compensation value, compensate, based on the obtained air pressure compensation value, the air pressure value detected by the first barometric pressure sensor 5a, and control the driving apparatus 6 based on a compensated air pressure value. The driving apparatus 6 is electrically connected to an air supply and exhaust apparatus 4, and is configured to drive, under control of the processor 7, the air supply and exhaust apparatus 4 to perform inflating or deflating.

In the blood pressure measurement device, when the air supply and exhaust apparatus 4 inflates/deflates the airbag 2, the second barometric pressure sensor 5b detects the air pressure value in the cavity 101, so that the processor 7 obtains an air pressure compensation value based on the air pressure value detected by the second barometric pressure sensor 5b and the stored correspondence between a cavity air pressure value and an air pressure compensation value, and then compensates, based on the obtained air pressure compensation value, the air pressure value detected by the first barometric pressure sensor 5a, to obtain an accurate air pressure value in the airbag 2. In this way, impact of the blood pressure measurement device on blood pressure measurement in an inflating/deflating process can be reduced, thereby improving blood pressure measurement accuracy.

In this application, because air fluctuation in the air cavity is caused by inflating/deflating of the air supply and exhaust apparatus 4, in an area closer to the air supply and exhaust apparatus 4, there is larger air pressure fluctuation. Therefore, the second barometric pressure sensor 5b may be disposed close to the air supply and exhaust apparatus 4, and a closer distance between the second barometric pressure sensor 5b and the air supply and exhaust apparatus 4 indicates a more accurate measurement result.

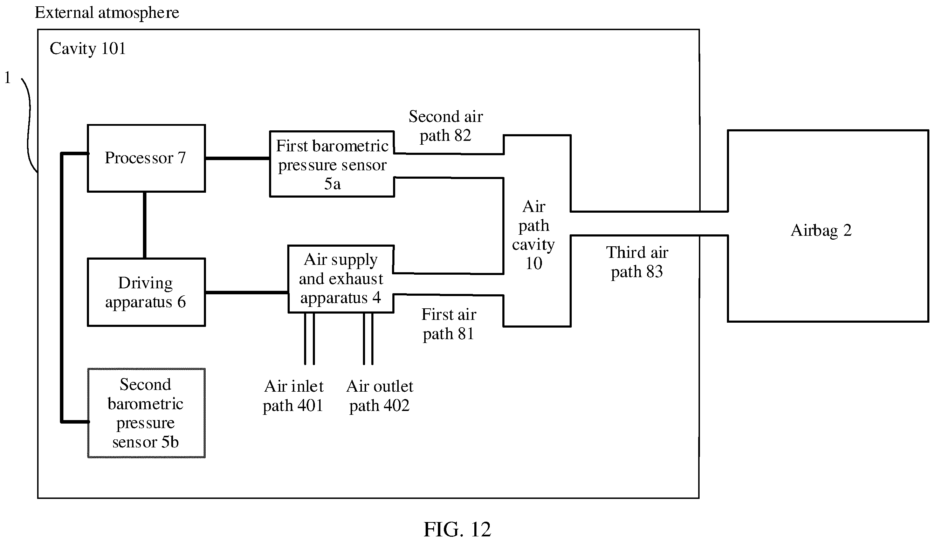

It should be noted that other structures of the blood pressure measurement device in the embodiment shown in FIG. 11 may be set with reference to any one of the foregoing embodiments. For example, as shown in FIG. 12, an air path cavity 10 may be disposed in the blood pressure measurement device, so that the air supply and exhaust apparatus 4 is connected to the air path cavity 10 through the first air path 81, the first barometric pressure sensor 5a may be connected to the air path cavity 10 through the second air path 82, and the air path cavity 10 is connected to the airbag 2 through the third air path 83, so as to implement a single-air nozzle connection between the body 1 and the airbag 2. Specific setting manners of other structures of the blood pressure measurement device shown in FIG. 11 and FIG. 12 are not described herein again.

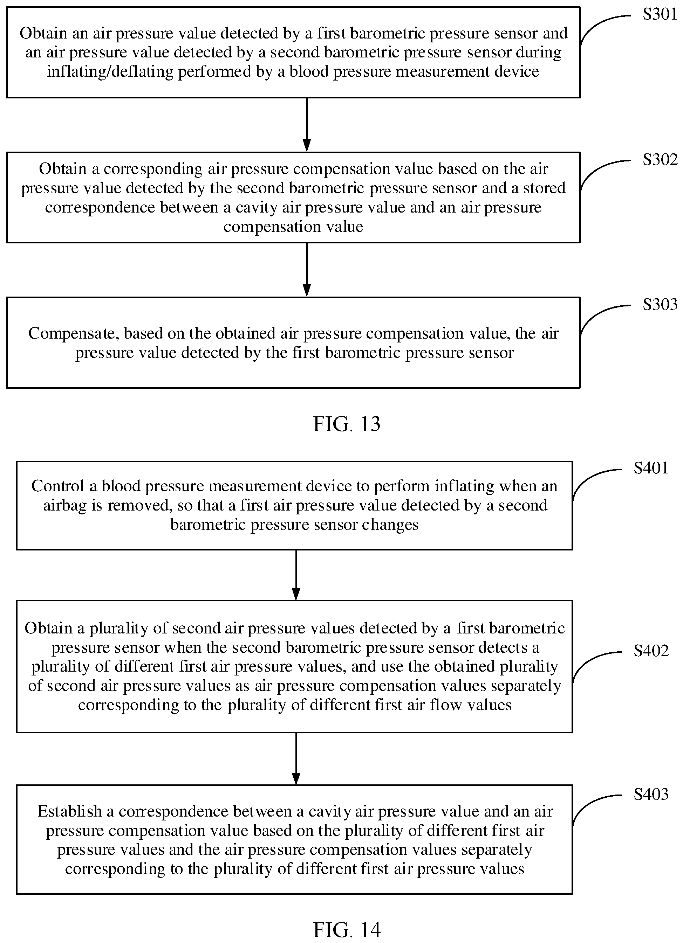

FIG. 13 shows a blood pressure measurement method of a blood pressure measurement device according to an embodiment of this application. The blood pressure measurement device mainly includes a body, an airbag, a first barometric pressure sensor, and a second barometric pressure sensor. The body includes a cavity, the barometric pressure sensor is located in the cavity, the airbag has an air cavity, and the air cavity is connected to the cavity. The first barometric pressure sensor is configured to detect an air pressure value in the air cavity, and the second barometric pressure sensor is configured to detect an air pressure value in the cavity. For specific implementation of the blood pressure measurement device, refer to the blood pressure measurement device in the embodiment shown in FIG. 11 and FIG. 12. Details are not described herein again. As shown in FIG. 13, the blood pressure measurement method of the blood pressure measurement device may include the following steps.

Step S301: Obtain an air pressure value detected by the first barometric pressure sensor and an air pressure value detected by the second barometric pressure sensor during inflating/deflating performed by the blood pressure measurement device.

Step S302: Obtain a corresponding air pressure compensation value based on the air pressure value detected by the second barometric pressure sensor and a stored correspondence between a cavity air pressure value and an air pressure compensation value.

Step S303: Compensate, based on the obtained air pressure compensation value, the air pressure value detected by the first barometric pressure sensor.

It can be learned that, during blood pressure measurement, the air pressure compensation value may be obtained based on the air pressure value detected by the second barometric pressure sensor and the stored correspondence between an air pressure value and an air pressure compensation value, and the air pressure value detected by the first barometric pressure sensor is compensated based on the obtained air pressure compensation value, to obtain an accurate air pressure value in the airbag. In this way, impact of air pressure in the cavity on blood pressure measurement can be reduced, thereby improving blood pressure measurement accuracy.

For example, the correspondence between a cavity air pressure value and an air pressure compensation value may alternatively be obtained in advance through data analysis, and is pre-stored in the processor or a memory in the blood pressure measurement device. The correspondence between a cavity air pressure value and an air pressure compensation value may be pre-established by using the processor before the blood pressure measurement device is delivered from a factory.

For example, as shown in FIG. 14, the processor may obtain the correspondence between a cavity air pressure value and an air pressure compensation value in the following manner.

Step S401: Control the blood pressure measurement device to perform inflating when the airbag is removed, so that a first air pressure value detected by the second barometric pressure sensor changes.

The airbag in the blood pressure measurement device is removed, so that the cavity of the body can be connected to the outside atmosphere, thereby ensuring that the air pressure value detected by the barometric pressure sensor is not affected by the air pressure in the air cavity of the airbag, and is mainly generated by air flow fluctuation in the cavity.

During specific implementation, the driving apparatus may be controlled to gradually increase from a smaller driving power to a maximum driving power to drive the air supply and exhaust apparatus.

Step S402: Obtain a plurality of second air pressure values detected by the first barometric pressure sensor when the second barometric pressure sensor detects a plurality of different first air pressure values, and use the obtained plurality of second air pressure values as air pressure compensation values separately corresponding to the plurality of different first air flow values.

During specific implementation, when the first air pressure value detected by the second barometric pressure sensor is q11, the second air pressure value q21 detected by the first barometric pressure sensor may be obtained, and q21 is used as an air pressure compensation value Δp1 corresponding to q11. Then, a driving state is increased, and when the first air pressure value detected by the second barometric pressure sensor is q12, a second air pressure value q22 detected by the first barometric pressure sensor is obtained, and q22 is used as an air pressure compensation value Δp2 corresponding to q12. By analogy, until the driving apparatus reaches a maximum driving state, a first air pressure value q1N detected by the second barometric pressure sensor is obtained, a second air pressure value q2N detected by the first barometric pressure sensor is obtained, and q2N is used as an air pressure compensation value ΔpN corresponding to q1N. Therefore, a correspondence between q11, q12, q13, q14, ..., and q1N and Δp1, Δp2, Δp3, Δp4, ..., and ΔpN may be established.

Step S403: establish a correspondence between a cavity air pressure value and an air pressure compensation value based on the plurality of different first air pressure values and the air pressure compensation values separately corresponding to the plurality of different first air pressure values.

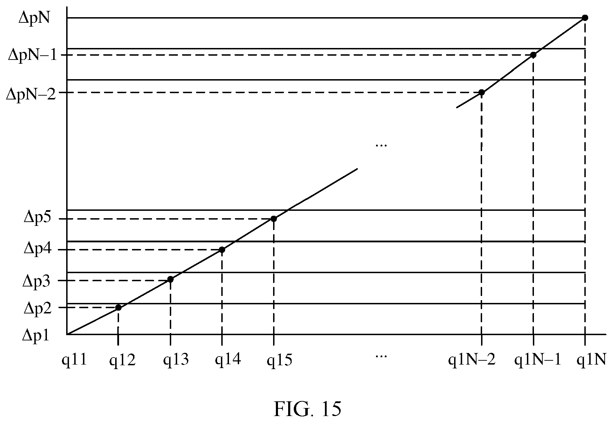

For example, as shown in FIG. 15, the processor may establish the correspondence between a cavity air pressure value and an air pressure compensation value in an interpolation method based on the plurality of different first air pressure values and the air pressure compensation values separately corresponding to the plurality of different first air pressure values. For example, for a first air pressure value q1(x) between q11 and q12, q12 and q13, ..., and q1N-1 and q1N, for example, q1(x) is located between q1i and q1j, an air pressure compensation value Δp(x) corresponding to q1(x) may be determined according to a formula Δp (x)=Δpi+[q1(x)-q1i](Δpj-Δpi)/(q1j-q1i), where i may be any number from 1 to N-1, and j=1+1.

During specific implementation, in this application, the air pressure compensation value Δp(x) corresponding to the first air pressure value q1(x) between 0 and q11, q11 and q12, q12 and q13, ..., and q1N-1 and q1N may alternatively be determined in another manner. This is not limited herein.

It can be learned from the foregoing that the pre-established correspondence between a cavity air pressure value and an air pressure compensation value is stored. In this way, during blood pressure detection, the air pressure compensation value may be obtained based on the air pressure value detected by the second barometric pressure sensor and the stored correspondence between a cavity air pressure value and an air pressure compensation value, and the air pressure value detected by the first barometric pressure sensor is compensated based on the obtained air pressure compensation value, to obtain an accurate air pressure value in the airbag. A process in which the blood pressure measurement device performs blood pressure measurement may include: controlling the driving apparatus to drive the air supply and exhaust apparatus to inflate the airbag, reading an air pressure value detected by the first barometric pressure sensor and an air pressure value detected by the second barometric pressure sensor, obtaining an air pressure compensation value based on the air pressure value detected by the second barometric pressure sensor and a stored correspondence between a cavity air pressure value and an air pressure compensation value, and compensating, based on the obtained air pressure compensation value, the air pressure value detected by the first barometric pressure sensor. Specifically, the obtained air pressure compensation value may be subtracted from the air pressure value detected by the first barometric pressure sensor, to obtain a compensated air pressure value. Then, an operating parameter (for example, a voltage, a current, or a frequency) of a driving circuit is controlled based on the compensated air pressure value to drive the air supply and exhaust apparatus to inflate the airbag. The foregoing procedure is performed at least once, for example, once, twice, three times, or four times, so that the compensated air pressure value meets a boost curve requirement for blood pressure measurement, thereby completing blood pressure measurement.

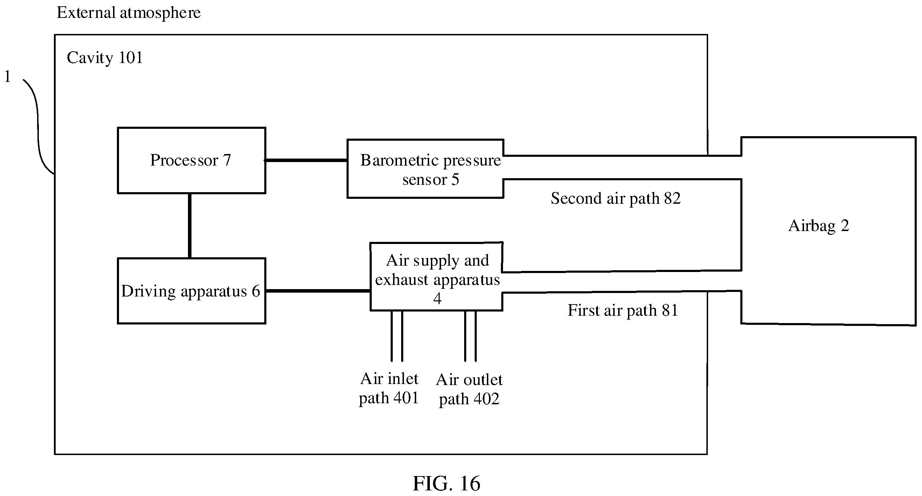

FIG. 16 is a schematic diagram of a structure of a blood pressure measurement device according to still another embodiment of this application. A structure of the blood pressure measurement device in this embodiment is also different from that in any one of the foregoing embodiments, and a main difference lies in that the blood pressure measurement device includes only one barometric pressure sensor and does not include a flowmeter. In the blood pressure measuring device, a barometric pressure sensor 5 is connected to an air cavity of an airbag 2 through a second air path 82, and is used to detect an air pressure value in the air cavity. A processor 7 is electrically connected to the barometric pressure sensor 5 and a driving apparatus 6, and is configured to: obtain an air pressure compensation value based on a driving state of the driving apparatus 6 and a correspondence that is between a driving state and an air pressure compensation value and that is stored in the blood pressure measurement device, compensate, based on the obtained air pressure compensation value, the air pressure value detected by the barometric pressure sensor 5, and control the driving apparatus 6 based on a compensated air pressure value. The driving apparatus 6 is electrically connected to an air supply and exhaust apparatus 4, and is configured to drive the air supply and exhaust apparatus 4 to perform inflating or deflating under control of the processor 7.

In the blood pressure measurement device, when the air supply and exhaust apparatus 4 inflates/deflates the airbag 2, the processor 7 may obtain the air pressure compensation value based on the driving state of the driving apparatus 6 and the stored correspondence between a driving state and an air pressure compensation value, and then compensate, based on the obtained air pressure compensation value, the air pressure value detected by the barometric pressure sensor, to obtain an accurate air pressure value in the airbag 2. In this way, impact of the blood pressure measurement device on blood pressure measurement in an inflating/deflating process can be reduced, thereby improving blood pressure measurement accuracy.

It should be noted that the driving state may include at least one parameter related to a supply power, for example, a driving voltage, a driving current, and a duty cycle.

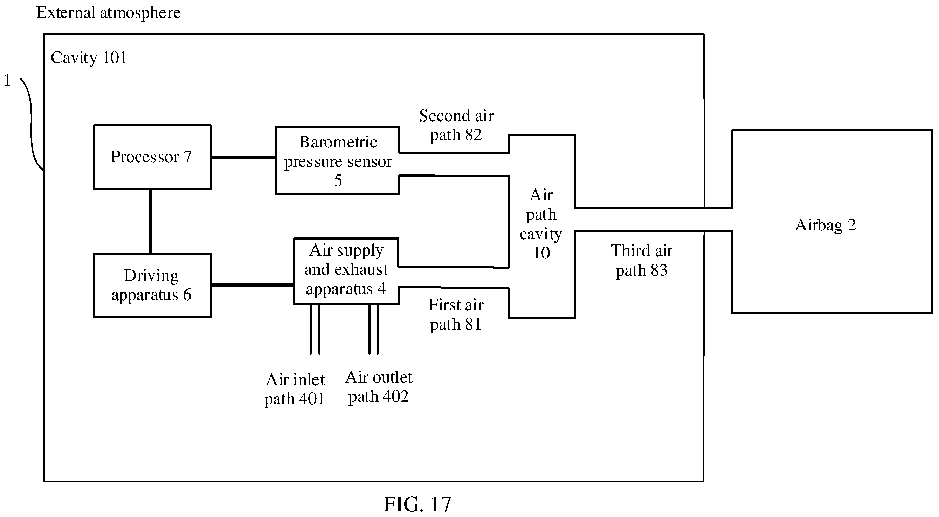

It should be noted that other structures of the blood pressure measurement device in the embodiment shown in FIG. 16 may be set with reference to any one of the foregoing embodiments. For example, as shown in FIG. 17, an air path cavity 10 may be disposed in the blood pressure measurement device, so that the air supply and exhaust apparatus 4 is connected to the air path cavity 10 through the first air path 81, the first barometric pressure sensor 5a may be connected to the air path cavity 10 through the second air path 82, and the air path cavity 10 is connected to the airbag 2 through the third air path 83, so as to implement a single-air nozzle connection between the body 1 and the airbag 2. Specific setting manners of other structures of the blood pressure measurement device shown in FIG. 16 and FIG. 17 are not described herein again.

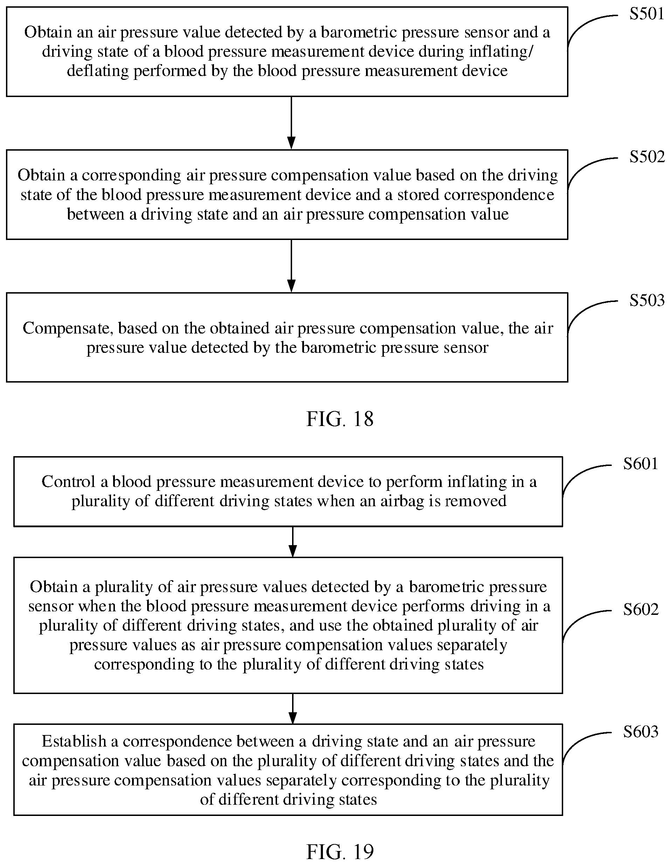

FIG. 18 shows a blood pressure measurement method of a blood pressure measurement device according to an embodiment of this application. The blood pressure measurement device mainly includes a body, an airbag, a barometric pressure sensor, and a flowmeter. The body includes a cavity, the barometric pressure sensor is located in the cavity, the airbag has an air cavity, and the air cavity is connected to the cavity. The barometric pressure sensor is configured to detect an air pressure value in the air cavity. For specific implementation of the blood pressure measurement device, refer to the blood pressure measurement device in the embodiments shown in FIG. 16 and FIG. 17. Details are not described herein again. As shown in FIG. 18, the blood pressure measurement method of the blood pressure measurement device may include the following steps.

S501: Obtain an air pressure value detected by the barometric pressure sensor and a driving state of the blood pressure measurement device during inflating/deflating performed by the blood pressure measurement device.

S502: Obtain a corresponding air pressure compensation value based on the driving state of the blood pressure measurement device and a stored correspondence between a driving state and an air pressure compensation value.

S503: Compensate, based on the obtained air pressure compensation value, the air pressure value detected by the barometric pressure sensor.

It can be learned that, during blood pressure measurement, the air pressure compensation value may be obtained based on the driving state of the blood pressure measurement device and the stored correspondence between a driving state and an air pressure compensation value, and the air pressure value detected by the barometric pressure sensor is compensated based on the obtained air pressure compensation value, to obtain an accurate air pressure value in the airbag. In this way, impact of air pressure in the cavity on blood pressure measurement can be reduced, thereby improving blood pressure measurement accuracy.

In this application, the correspondence between a driving state and an air pressure compensation value may be obtained in advance by analyzing pre-tested data, and is pre-stored in the processor or a memory of the blood pressure measurement device.

For example, in this application, as shown in FIG. 19, the processor may obtain the correspondence between a driving state and an air pressure compensation value in the following manner.

Step S601: Control the blood pressure measurement device to perform inflating in a plurality of different driving states when the airbag is removed.

The airbag in the blood pressure measurement device is removed, so that the cavity of the body can be connected to the outside atmosphere, thereby ensuring that the air pressure value detected by the barometric pressure sensor is not affected by the air pressure in the air cavity of the airbag, and is mainly generated by air flow fluctuation in the cavity.

During specific implementation, the driving apparatus may be controlled to gradually increase from a smaller driving power to a maximum driving power to drive the air supply and exhaust apparatus 4.

Step S602: Obtain a plurality of air pressure values detected by the barometric pressure sensor when the blood pressure measurement device performs driving in a plurality of different driving states, and use the obtained plurality of air pressure values as air pressure compensation values separately corresponding to the plurality of different driving states.

During specific implementation, when the driving power is S1, an air pressure value q1 detected by the barometric pressure sensor may be obtained, and q1 is used as an air pressure compensation value Δp1 corresponding to S1. Then, when the driving power is increased to S2, an air pressure value q2 detected by the barometric pressure sensor is obtained, and q2 is used as an air pressure compensation value Δp2 corresponding to S2. By analogy, until the driving apparatus reaches a maximum driving power SN, an air pressure value qN detected by the barometric pressure sensor is obtained, and qN is used as an air pressure compensation value ΔpN corresponding to SN. Therefore, a correspondence between S1, S2, S3, S4, ..., and SN and Δp1, Δp2, Δp3, Δp4, ..., and ΔpN may be established.

Step S603: Establish a correspondence between a driving state and an air pressure compensation value based on the plurality of different driving states and the air pressure compensation values separately corresponding to the plurality of different driving states.

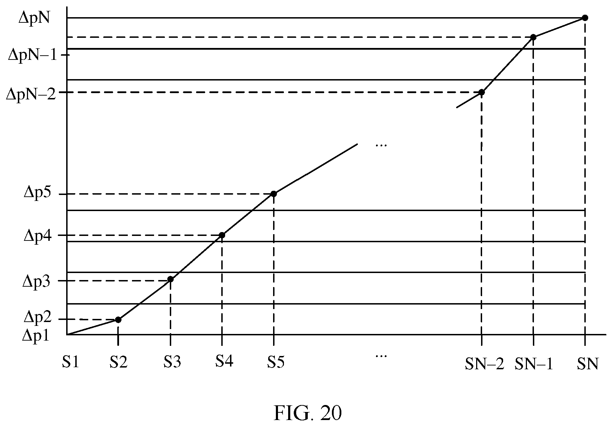

For example, as shown in FIG. 20, the processor may establish the correspondence between a driving state and an air pressure compensation value in an interpolation method based on the plurality of different driving states and the air pressure compensation values separately corresponding to the plurality of different driving states. For example, the driving state is a driving power. For example, for a driving power S(x) between S1 and S2, S2 and S3, ..., and SN-1 and SN, for example, S(x) is located between Si and Sj, an air pressure compensation value Δp(x) corresponding to S(x) may be determined according to a formula Δp(x)=Δpi+[S(x)-Si](Δpj-Δpi)/(Sj-Si). i may be any number from 1 to N-1, and j=1+1.

During specific implementation, in this application, the correspondence between a driving state and an air pressure compensation value may be established in another manner based on correspondences between S1, S2, S3, S4, ..., and SN and Δp1, Δp2, Δp3, Δp4, ..., and ΔpN. This is not limited herein.

It can be learned from the foregoing that the pre-established correspondence between a driving state and an air pressure compensation value is stored. In this way, during blood pressure detection, the air pressure compensation value may be obtained based on the driving state and the stored correspondence between a driving state and an air pressure compensation value, and the air pressure value detected by the barometric pressure sensor is compensated based on the obtained air pressure compensation value, to obtain an accurate air pressure value in the airbag. A process in which the blood pressure measurement device performs blood pressure measurement may include: controlling the driving apparatus to drive the air supply and exhaust apparatus to inflate the airbag, reading an air pressure value detected by the barometric pressure sensor, obtaining an air pressure compensation value based on a driving state of the driving apparatus and a stored correspondence between a driving state and an air pressure compensation value, and compensating, based on the obtained air pressure compensation value, the air pressure value detected by the barometric pressure sensor. Specifically, the obtained air pressure compensation value may be subtracted from the air pressure value detected by the barometric pressure sensor, to obtain a compensated air pressure value. Then, an operating parameter (for example, a voltage, a current, or a frequency) of a driving circuit is controlled based on the compensated air pressure value, to drive the air supply and exhaust apparatus to inflate the airbag. The foregoing procedure is performed at least once, for example, once, twice, three times, or four times, so that the compensated air pressure value meets a boost curve requirement for blood pressure measurement, thereby completing blood pressure measurement.

In conclusion, when the blood pressure measurement device provided in this application is used to perform blood pressure measurement, an air pressure value detected by the barometric pressure sensor may be compensated based on an obtained air pressure compensation value, so that impact of air pressure in the cavity of the body of the blood pressure measurement device on a measurement value of the barometric pressure sensor can be effectively reduced, and a blood pressure measurement result is accurate.

Correspondingly, an embodiment of this application further provides an electronic device. The electronic device may include any blood pressure measurement device provided in the foregoing embodiments. A problem-resolving principle of the electronic device is similar to that of the foregoing blood pressure measurement device. Therefore, for implementation of the electronic device, refer to implementation of the foregoing blood pressure measurement device, and details are not described again.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. A blood pressure measurement device, comprising: a body (1), a processor (7), an airbag (2), an air supply and exhaust apparatus (4), a driving apparatus (6), a barometric pressure sensor (5), and a flowmeter (9), wherein
the body (1) comprises a cavity (101), the cavity (101) is enclosed by a plurality of side walls, and the air supply and exhaust apparatus (4) and the barometric pressure sensor (5) are both disposed in the cavity (101);
the airbag (2) has an air cavity, and the airbag (2) is fastened to an end of the body (1);
the air supply and exhaust apparatus (4) is connected to the air cavity of the airbag (2) through a first air path (81);
the barometric pressure sensor (5) is connected to the air cavity of the airbag (2) through a second air path (82), and is configured to detect an air pressure value in the air cavity;
the flowmeter (9) is configured to detect an air flow value between the air supply and exhaust apparatus (4) and the airbag (2), or is configured to detect an air flow value between the barometric pressure sensor (5) and the airbag (2);
the processor (7) is electrically connected to the barometric pressure sensor (5), the flowmeter (9), and the driving apparatus (6), and is configured to: obtain a corresponding air pressure compensation value based on the air flow value detected by the flowmeter (9) and a correspondence that is between an air flow value and an air pressure compensation value and that is stored in the blood pressure measurement device, compensate, based on the obtained air pressure compensation value, the air pressure value detected by the barometric pressure sensor (5), and control the driving apparatus (6) based on a compensated air pressure value; and
the driving apparatus (6) is electrically connected to the air supply and exhaust apparatus (4), and is configured to drive, under control of the processor (7), the air supply and exhaust apparatus (4) to perform inflating or deflating,
wherein the blood pressure measurement device further comprises an air path cavity (10), and the air path cavity (10) is disposed in the cavity (101) of the body (1); and
the air supply and exhaust apparatus (4) is connected to the air path cavity (10) through the first air path (81), the barometric pressure sensor (5) is connected to the air path cavity (10) through the second air path (82), and the air path cavity (10) is connected to the air cavity of the airbag (2) through a third air path (83).

2. The blood pressure measurement device according to claim 1, wherein that the flowmeter (9) detects an air flow value between the air supply and exhaust apparatus (4) and the airbag (2) is specifically:
the flowmeter (9) is located in the first air path (81), and is configured to detect an air flow value in the first air path (81); and
that the flowmeter (9) detects an air flow value between the barometric pressure sensor (5) and the airbag (2) is specifically:
the flowmeter (9) is located in the second air path (82), and is configured to detect an air flow value in the second air path (82).

3. The blood pressure measurement device according to claim 1, wherein the flowmeter (9) is located in the third air path, and is configured to detect an air flow value in the third air path.

4. The blood pressure measurement device according to any one of claims 1 to 3, wherein the processor (7) is further configured to:
control the blood pressure measurement device to perform inflating when the airbag (2) is removed, so that the air flow value detected by the flowmeter (9) changes;
obtain a plurality of air pressure values detected by the barometric pressure sensor (5) when the flowmeter (9) detects a plurality of different air flow values, and use the obtained plurality of air pressure values as air pressure compensation values separately corresponding to the plurality of different air flow values; and
establish the correspondence between an air flow value and an air pressure compensation value based on the plurality of different air flow values and the air pressure compensation values separately corresponding to the plurality of different air flow values.

5. The blood pressure measurement device according to claim 4, wherein when establishing the correspondence between an air flow value and an air pressure compensation value based on the plurality of different air flow values and the air pressure compensation values separately corresponding to the plurality of different air flow values, the processor (7) is specifically configured to:
establish the correspondence between an air flow value and an air pressure compensation value in an interpolation method based on the plurality of different air flow values and the air pressure compensation values separately corresponding to the plurality of different air flow values.

6. A blood pressure measurement device, comprising: a body (1), a processor (7), an airbag (2), an air supply and exhaust apparatus (4), a driving apparatus (6), a first barometric pressure sensor (5a), and a second barometric pressure sensor (5b), wherein
the body (1) comprises a cavity (101), the cavity (101) is enclosed by a plurality of side walls, and the air supply and exhaust apparatus (4), the first barometric pressure sensor (5a), and the second barometric pressure sensor (5b) are all disposed in the cavity (101);
the airbag (2) has an air cavity, and the airbag (2) is fastened to an end of the body (1);
the air supply and exhaust apparatus (4) is connected to the air cavity of the airbag (2) through a first air path (81);
the first barometric pressure sensor (5a) is connected to the air cavity of the airbag (2) through a second air path (82), and is configured to detect an air pressure value in the air cavity;
the second barometric pressure sensor (5b) is configured to detect an air pressure value in the cavity (101);
the processor (7) is electrically connected to the first barometric pressure sensor (5a), the second barometric pressure sensor (5b), and the driving apparatus (6), and is configured to: obtain a corresponding air pressure compensation value based on the air pressure value detected by the second barometric pressure sensor (5b) and a correspondence that is between a cavity air pressure value and an air pressure compensation value and that is stored in the blood pressure measurement device, compensate, based on the obtained air pressure compensation value, the air pressure value detected by the first barometric pressure sensor (5a), and control the driving apparatus (6) based on a compensated air pressure value; and
the driving apparatus (6) is electrically connected to the air supply and exhaust apparatus (4), and is configured to drive, under control of the processor (7), the air supply and exhaust apparatus (4) to perform inflating or deflating,
wherein the blood pressure measurement device further comprises an air path cavity (10), and the air path cavity (10) is disposed in the cavity (101) of the body (1); and
the air supply and exhaust apparatus (4) is connected to the air path cavity (10) through the first air path (81), the first barometric pressure sensor (5a) is connected to the air path cavity (10) through the second air path (82), and the air path cavity (10) is connected to the air cavity of the airbag (2) through a third air path (83).

7. The blood pressure measurement device according to claim 6, wherein the second barometric pressure sensor (5b) is disposed close to the air supply and exhaust apparatus (4).

8. The blood pressure measurement device according to any one of claims 6 or 7, wherein the processor (7) is further configured to:
control the blood pressure measurement device to perform inflating when the airbag (2) is removed, so that a first air pressure value detected by the second barometric pressure sensor (5b) changes;
obtain a plurality of second air pressure values detected by the first barometric pressure sensor (5a) when the second barometric pressure sensor (5b) detects a plurality of different first air pressure values, and use the obtained plurality of second air pressure values as air pressure compensation values separately corresponding to the plurality of different first air pressure values; and
establish the correspondence between a cavity air pressure value and an air pressure compensation value based on the plurality of different first air pressure values and the air pressure compensation values separately corresponding to the plurality of different first air pressure values.

9. The blood pressure measurement device according to claim 8, wherein when establishing the correspondence between a cavity air pressure value and an air pressure compensation value based on the plurality of different first air pressure values and the air pressure compensation values separately corresponding to the plurality of different first air pressure values, the processor (7) is specifically configured to:
establish the correspondence between a cavity air pressure value and an air pressure compensation value in an interpolation method based on the plurality of different first air pressure values and the air pressure compensation values separately corresponding to the plurality of different first air pressure values.

10. A blood pressure measurement device, comprising: a body (1), a processor (7), an airbag (2), an air supply and exhaust apparatus (4), a driving apparatus (6), and a barometric pressure sensor (5), wherein
the body (1) comprises a cavity (101), the cavity (101) is enclosed by a plurality of side walls, and the air supply and exhaust apparatus (4) and the barometric pressure sensor (5) are both disposed in the cavity (101);
the airbag (2) has an air cavity, and the airbag (2) is fastened to an end of the body (1);
the air supply and exhaust apparatus (4) is connected to the air cavity of the airbag (2) through a first air path (81);
the barometric pressure sensor is connected to the air cavity of the airbag (2) through a second air path (82), and is configured to detect an air pressure value in the air cavity;
the processor (7) is electrically connected to the barometric pressure sensor (5) and the driving apparatus (6), and is configured to: obtain a corresponding air pressure compensation value based on a driving state of the driving apparatus (6) and a correspondence that is between a driving state and an air pressure compensation value and that is stored in the blood pressure measurement device, compensate, based on the obtained air pressure compensation value, an air pressure value detected by the barometric pressure sensor (5), and control the driving apparatus (6) based on a compensated air pressure value; and
the driving apparatus (6) is electrically connected to the air supply and exhaust apparatus (4), and is configured to drive, under control of the processor (7), the air supply and exhaust apparatus (4) to perform inflating or deflating,
wherein the blood pressure measurement device further comprises an air path cavity (10), and the air path cavity (10) is disposed in the cavity (101) of the body (1); and
the air supply and exhaust apparatus (4) is connected to the air path cavity (10) through the first air path (81), the barometric pressure sensor (5) is connected to the air path cavity (10) through the second air path (82), and the air path cavity (10) is connected to the air cavity of the airbag (2) through a third air path (83).

11. The blood pressure measurement device according to claim 10, wherein the processor (7) is further configured to:
control the blood pressure measurement device to perform inflating in a plurality of different driving states when the airbag (2) is removed;
obtain a plurality of air pressure values detected by the barometric pressure sensor (5) when the blood pressure measurement device performs inflating in the plurality of different driving states, and use the obtained plurality of air pressure values as air pressure compensation values separately corresponding to the plurality of different driving states; and
establish the correspondence between a driving state and an air pressure compensation value based on the plurality of different driving states and the air pressure compensation values separately corresponding to the plurality of different driving states.

12. The blood pressure measurement device according to claim 11, wherein when establishing the correspondence between a driving state and an air pressure compensation value based on the plurality of different driving states and the air pressure compensation values separately corresponding to the plurality of different driving states, the processor (7) is specifically configured to:
establish the correspondence between a driving state and an air pressure compensation value in an interpolation method based on the plurality of different driving states and the air pressure compensation values separately corresponding to the plurality of different driving states.

13. An electronic device, comprising the blood pressure measurement device according to any one of claims 1 to 12.

## Patentansprüche

1. Blutdruckmessvorrichtung, umfassend: ein Gehäuse (1), einen Prozessor (7), ein Luftkissen (2), eine Luftzufuhr- und - ablassvorrichtung (4), eine Antriebsvorrichtung (6), einen barometrischen Drucksensor (5) und einen Durchflussmesser (9), wobei
das Gehäuse (1) einen Hohlraum (101) umfasst, der Hohlraum (101) von einer Vielzahl von Seitenwänden umschlossen ist und die Luftzufuhr- und -ablassvorrichtung (4) und der barometrische Drucksensor (5) beide im Hohlraum (101) angeordnet sind;
das Luftkissen (2) einen Lufthohlraum aufweist und das Luftkissen (2) an einem Ende des Gehäuses (1) befestigt ist;
die Luftzufuhr- und -ablassvorrichtung (4) über einen ersten Luftweg (81) mit dem Lufthohlraum des Luftkissens (2) verbunden ist;
der barometrische Drucksensor (5) über einen zweiten Luftweg (82) mit dem Lufthohlraum des Luftkissens (2) verbunden und zum Erfassen eines Luftdruckwerts im Lufthohlraum konfiguriert ist; der Durchflussmesser (9) zum Erfassen eines Luftdurchflusswerts zwischen der Luftzufuhr- und -ablassvorrichtung (4) und dem Luftkissen (2) konfiguriert ist oder zum Erfassen eines Luftdurchflusswerts zwischen dem barometrischen Drucksensor (5) und dem Luftkissen (2) konfiguriert ist;
der Prozessor (7) elektrisch mit dem barometrischen Drucksensor (5), dem Durchflussmesser (9) und der Antriebsvorrichtung (6) verbunden ist und zu Folgendem konfiguriert ist: Erhalten eines entsprechenden Luftdruckkompensationswerts basierend auf dem vom Durchflussmesser (9) erfassten Luftdurchflusswert und einer Entsprechung, die zwischen einem Luftdurchflusswert und einem Luftdruckkompensationswert besteht und die in der Blutdruckmessvorrichtung gespeichert ist, Kompensieren des vom barometrischen Drucksensor (5) erfassten Luftdruckwerts basierend auf dem erhaltenen Luftdruckkompensationswert und Steuern der Antriebsvorrichtung (6) basierend auf einem kompensierten Luftdruckwert; und
die Antriebsvorrichtung (6) elektrisch mit der Luftzufuhr- und -ablassvorrichtung (4) verbunden ist und zum Antreiben der Luftzufuhr- und -ablassvorrichtung (4) unter Steuerung des Prozessors (7) konfiguriert ist, um ein Aufblasen oder Entleeren durchzuführen,
wobei die Blutdruckmessvorrichtung ferner einen Luftweghohlraum (10) umfasst und der Luftweghohlraum (10) im Hohlraum (101) des Gehäuses (1) angeordnet ist; und
die Luftzufuhr- und -ablassvorrichtung (4) über den ersten Luftweg (81) mit dem Luftweghohlraum (10) verbunden ist, der barometrische Drucksensor (5) über den zweiten Luftweg (82) mit dem Luftweghohlraum (10) verbunden ist und der Luftweghohlraum (10) über einen dritten Luftweg (83) mit dem Lufthohlraum des Luftkissens (2) verbunden ist.

2. Blutdruckmessvorrichtung nach Anspruch 1, wobei das Erfassen eines Luftdurchflusswerts zwischen der Luftzufuhr- und - ablassvorrichtung (4) und dem Luftkissen (2) durch den Durchflussmesser (9) spezifisch folgendermaßen erfolgt:
der Durchflussmesser (9) ist im ersten Luftweg (81) positioniert und ist zum Erfassen eines Luftdurchflusswerts im ersten Luftweg (81) konfiguriert; und
das Erfassen eines Luftdurchflusswerts zwischen dem barometrischen Drucksensor (5) und dem Luftkissen (2) durch den Durchflussmesser (9) spezifisch folgendermaßen erfolgt:
der Durchflussmesser (9) ist im zweiten Luftweg (82) positioniert und ist zum Erfassen eines Luftdurchflusswerts im zweiten Luftweg (82) konfiguriert.

3. Blutdruckmessvorrichtung nach Anspruch 1, wobei der Durchflussmesser (9) im dritten Luftweg positioniert ist und zum Erfassen eines Luftdurchflusswerts im dritten Luftweg konfiguriert ist.

4. Blutdruckmessvorrichtung nach einem der Ansprüche 1 bis 3, wobei der Prozessor (7) ferner zu Folgendem konfiguriert ist:
Steuern der Blutdruckmessvorrichtung, um das Aufblasen durchzuführen, wenn das Luftkissen (2) entfernt ist, sodass sich der vom Durchflussmesser (9) erfasste Luftdurchflusswert ändert;
Erhalten einer Vielzahl von Luftdruckwerten, die vom barometrischen Drucksensor (5) erfasst werden, wenn der Durchflussmesser (9) eine Vielzahl von unterschiedlichen Luftdurchflusswerten erfasst, und Verwenden der erhaltenen Vielzahl von Luftdruckwerten als Luftdruckkompensationswerte, die separat der Vielzahl von unterschiedlichen Luftdurchflusswerten entsprechen; und
Herstellen der Entsprechung zwischen einem Luftdurchflusswert und einem Luftdruckkompensationswert basierend auf der Vielzahl von unterschiedlichen Luftdurchflusswerten und den Luftdruckkompensationswerten, die separat der Vielzahl von unterschiedlichen Luftdurchflusswerten entsprechen.

5. Blutdruckmessvorrichtung nach Anspruch 4, wobei der Prozessor (7) beim Herstellen der Entsprechung zwischen einem Luftdurchflusswert und einem Luftdruckkompensationswert basierend auf der Vielzahl von unterschiedlichen Luftdurchflusswerten und den Luftdruckkompensationswerten, die separat der Vielzahl von unterschiedlichen Luftdurchflusswerten entsprechen, speziell zu Folgendem konfiguriert ist:
Herstellen der Entsprechung zwischen einem Luftdurchflusswert und einem Luftdruckkompensationswert in einem Interpolationsverfahren basierend auf der Vielzahl von unterschiedlichen Luftdurchflusswerten und den Luftdruckkompensationswerten, die separat der Vielzahl von unterschiedlichen Luftdurchflusswerten entsprechen.

6. Blutdruckmessvorrichtung, umfassend: ein Gehäuse (1), einen Prozessor (7), ein Luftkissen (2), eine Luftzufuhr- und - ablassvorrichtung (4), eine Antriebsvorrichtung (6), einen ersten barometrischen Drucksensor (5a) und einen zweiten barometrischen Drucksensor (5b), wobei
das Gehäuse (1) einen Hohlraum (101) umfasst, der Hohlraum (101) von einer Vielzahl von Seitenwänden umschlossen ist und die Luftzufuhr- und -ablassvorrichtung (4), der erste barometrische Drucksensor (5a) und der zweite barometrische Drucksensor (5b) alle im Hohlraum (101) angeordnet sind;
das Luftkissen (2) einen Lufthohlraum aufweist und das Luftkissen (2) an einem Ende des Gehäuses (1) befestigt ist;
die Luftzufuhr- und -ablassvorrichtung (4) über einen ersten Luftweg (81) mit dem Lufthohlraum des Luftkissens (2) verbunden ist;
der erste barometrische Drucksensor (5a) über einen zweiten Luftweg (82) mit dem Lufthohlraum des Luftkissens (2) verbunden und zum Erfassen eines Luftdruckwerts im Lufthohlraum konfiguriert ist;
der zweite barometrische Drucksensor (5b) zum Erfassen eines Luftdruckwerts im Hohlraum (101) konfiguriert ist;
der Prozessor (7) elektrisch mit dem ersten barometrischen Drucksensor (5a), dem zweiten barometrischen Drucksensor (5b) und der Antriebsvorrichtung (6) verbunden ist und zu Folgendem konfiguriert ist: Erhalten eines entsprechenden Luftdruckkompensationswerts basierend auf dem vom zweiten barometrischen Drucksensor (5b) erfassten Luftdruckwert und einer Entsprechung, die zwischen einem Hohlraumluftdruckwert und einem Luftdruckkompensationswert besteht und die in der Blutdruckmessvorrichtung gespeichert ist, Kompensieren des vom ersten barometrischen Drucksensor (5a) erfassten Luftdruckwerts basierend auf dem erhaltenen Luftdruckkompensationswert und Steuern der Antriebsvorrichtung (6) basierend auf einem kompensierten Luftdruckwert; und
die Antriebsvorrichtung (6) elektrisch mit der Luftzufuhr- und -ablassvorrichtung (4) verbunden ist und zum Antreiben der Luftzufuhr- und -ablassvorrichtung (4) unter Steuerung des Prozessors (7) konfiguriert ist, um ein Aufblasen oder Entleeren durchzuführen,
wobei die Blutdruckmessvorrichtung ferner einen Luftweghohlraum (10) umfasst und der Luftweghohlraum (10) im Hohlraum (101) des Gehäuses (1) angeordnet ist; und
die Luftzufuhr- und -ablassvorrichtung (4) über den ersten Luftweg (81) mit dem Luftweghohlraum (10) verbunden ist, der erste barometrische Drucksensor (5a) über den zweiten Luftweg (82) mit dem Luftweghohlraum (10) verbunden ist und der Luftweghohlraum (10) über einen dritten Luftweg (83) mit dem Lufthohlraum des Luftkissens (2) verbunden ist.

7. Blutdruckmessvorrichtung nach Anspruch 6, wobei der zweite barometrische Drucksensor (5b) in der Nähe der Luftzufuhr- und -ablassvorrichtung (4) angeordnet ist.

8. Blutdruckmessvorrichtung nach einem der Ansprüche 6 oder 7, wobei der Prozessor (7) ferner zu Folgendem konfiguriert ist:
Steuern der Blutdruckmessvorrichtung, um das Aufblasen durchzuführen, wenn das Luftkissen (2) entfernt ist, sodass sich ein vom zweiten barometrischen Drucksensor (5b) erfasster erster Luftdruckwert ändert;
Erhalten einer Vielzahl von zweiten Luftdruckwerten, die vom ersten barometrischen Drucksensor (5a) erfasst werden, wenn der zweite barometrische Drucksensor (5b) eine Vielzahl von unterschiedlichen ersten Luftdruckwerten erfasst, und Verwenden der erhaltenen Vielzahl von zweiten Luftdruckwerten als Luftdruckkompensationswerte, die separat der Vielzahl von unterschiedlichen ersten Luftdruckwerten entsprechen; und
Herstellen der Entsprechung zwischen einem Hohlraumluftdruckwert und einem Luftdruckkompensationswert basierend auf der Vielzahl von unterschiedlichen ersten Luftdruckwerten und den Luftdruckkompensationswerten, die separat der Vielzahl von unterschiedlichen ersten Luftdruckwerten entsprechen.

9. Blutdruckmessvorrichtung nach Anspruch 8, wobei der Prozessor (7) beim Herstellen der Entsprechung zwischen einem Hohlraumluftdruckwert und einem Luftdruckkompensationswert basierend auf der Vielzahl von unterschiedlichen ersten Luftdruckwerten und den Luftdruckkompensationswerten, die separat der Vielzahl von unterschiedlichen ersten Luftdruckwerten entsprechen, speziell zu Folgendem konfiguriert ist:
Herstellen der Entsprechung zwischen einem Hohlraumluftdruckwert und einem Luftdruckkompensationswert in einem Interpolationsverfahren basierend auf der Vielzahl von unterschiedlichen ersten Luftdruckwerten und den Luftdruckkompensationswerten, die separat der Vielzahl von unterschiedlichen ersten Luftdruckwerten entsprechen.

10. Blutdruckmessvorrichtung, umfassend: ein Gehäuse (1), einen Prozessor (7), ein Luftkissen (2), eine Luftzufuhr- und - ablassvorrichtung (4), eine Antriebsvorrichtung (6) und einen barometrischen Drucksensor (5), wobei
das Gehäuse (1) einen Hohlraum (101) umfasst, der Hohlraum (101) von einer Vielzahl von Seitenwänden umschlossen ist und die Luftzufuhr- und -ablassvorrichtung (4) und der barometrische Drucksensor (5) beide im Hohlraum (101) angeordnet sind;
das Luftkissen (2) einen Lufthohlraum aufweist und das Luftkissen (2) an einem Ende des Gehäuses (1) befestigt ist;
die Luftzufuhr- und -ablassvorrichtung (4) über einen ersten Luftweg (81) mit dem Lufthohlraum des Luftkissens (2) verbunden ist;
der barometrische Drucksensor über einen zweiten Luftweg (82) mit dem Lufthohlraum des Luftkissens (2) verbunden und zum Erfassen eines Luftdruckwerts im Lufthohlraum konfiguriert ist; der Prozessor (7) elektrisch mit dem barometrischen Drucksensor (5) und der Antriebsvorrichtung (6) verbunden ist und zu Folgendem konfiguriert ist: Erhalten eines entsprechenden Luftdruckkompensationswerts basierend auf einem Antriebszustand der Antriebsvorrichtung (6) und einer Entsprechung, die zwischen einem Antriebszustand und einem Luftdruckkompensationswert besteht und die in der Blutdruckmessvorrichtung gespeichert ist, Kompensieren eines vom barometrischen Drucksensor (5) erfassten Luftdruckwerts basierend auf dem erhaltenen Luftdruckkompensationswert und Steuern der Antriebsvorrichtung (6) basierend auf einem kompensierten Luftdruckwert; und
die Antriebsvorrichtung (6) elektrisch mit der Luftzufuhr- und -ablassvorrichtung (4) verbunden ist und zum Antreiben der Luftzufuhr- und -ablassvorrichtung (4) unter Steuerung des Prozessors (7) konfiguriert ist, um ein Aufblasen oder Entleeren durchzuführen,
wobei die Blutdruckmessvorrichtung ferner einen Luftweghohlraum (10) umfasst und der Luftweghohlraum (10) im Hohlraum (101) des Gehäuses (1) angeordnet ist; und
die Luftzufuhr- und -ablassvorrichtung (4) über den ersten Luftweg (81) mit dem Luftweghohlraum (10) verbunden ist, der barometrische Drucksensor (5) über den zweiten Luftweg (82) mit dem Luftweghohlraum (10) verbunden ist und der Luftweghohlraum (10) über einen dritten Luftweg (83) mit dem Lufthohlraum des Luftkissens (2) verbunden ist.

11. Blutdruckmessvorrichtung nach Anspruch 10, wobei der Prozessor (7) ferner zu Folgendem konfiguriert ist:
Steuern der Blutdruckmessvorrichtung, um das Aufblasen in einer Vielzahl von unterschiedlichen Antriebszuständen durchzuführen, wenn das Luftkissen (2) entfernt ist;
Erhalten einer Vielzahl von Luftdruckwerten, die vom barometrischen Drucksensor (5) erfasst werden, wenn die Blutdruckmessvorrichtung das Aufblasen in der Vielzahl von unterschiedlichen Antriebszuständen durchführt, und Verwenden der erhaltenen Vielzahl von Luftdruckwerten als Luftdruckkompensationswerte, die separat der Vielzahl von unterschiedlichen Antriebszuständen entsprechen; und
Herstellen der Entsprechung zwischen einem Antriebszustand und einem Luftdruckkompensationswert basierend auf der Vielzahl von unterschiedlichen Antriebszuständen und den Luftdruckkompensationswerten, die separat der Vielzahl von unterschiedlichen Antriebszuständen entsprechen.

12. Blutdruckmessvorrichtung nach Anspruch 11, wobei der Prozessor (7) beim Herstellen der Entsprechung zwischen einem Antriebszustand und einem Luftdruckkompensationswert basierend auf der Vielzahl von unterschiedlichen Antriebszuständen und den Luftdruckkompensationswerten, die separat der Vielzahl von unterschiedlichen Antriebszuständen entsprechen, speziell zu Folgendem konfiguriert ist:
Herstellen der Entsprechung zwischen einem Antriebszustand und einem Luftdruckkompensationswert in einem Interpolationsverfahren basierend auf der Vielzahl von unterschiedlichen Antriebszuständen und den Luftdruckkompensationswerten, die separat der Vielzahl von unterschiedlichen Antriebszuständen entsprechen.

13. Elektronische Vorrichtung, umfassend die Blutdruckmessvorrichtung nach einem der Ansprüche 1 bis 12.

## Revendications

1. Dispositif de mesure de pression artérielle, comprenant : un corps (1), un processeur (7), une poche gonflable (2), un appareil d'alimentation et d'évacuation d'air (4), un appareil d'entraînement (6), un capteur de pression barométrique (5) et un débitmètre (9), dans lequel
le corps (1) comprend une cavité (101), la cavité (101) est entourée d'une pluralité de parois latérales, et l'appareil d'alimentation et d'évacuation d'air (4) et le capteur de pression barométrique (5) sont tous deux disposés dans la cavité (101) ;
la poche gonflable (2) a une cavité d'air, et la poche gonflable (2) est fixée à une extrémité du corps (1) ;
l'appareil d'alimentation et d'évacuation d'air (4) est connecté à la cavité d'air de la poche gonflable (2) via un premier trajet d'air (81) ;
le capteur de pression barométrique (5) est connecté à la cavité d'air de la poche gonflable (2) via un deuxième trajet d'air (82) et est configuré pour détecter une valeur de pression d'air dans la cavité d'air ;
le débitmètre (9) est configuré pour détecter une valeur de débit d'air entre l'appareil d'alimentation et d'évacuation d'air (4) et la poche gonflable (2), ou est configuré pour détecter une valeur de débit d'air entre le capteur de pression barométrique (5) et la poche gonflable (2) ;
le processeur (7) est connecté électriquement au capteur de pression barométrique (5), au débitmètre (9) et à l'appareil d'entraînement (6), et est configuré pour : obtenir une valeur de compensation de pression d'air correspondante sur la base de la valeur de débit d'air détectée par le débitmètre (9) et d'une correspondance entre une valeur de débit d'air et une valeur de compensation de pression d'air qui est stockée dans le dispositif de mesure de pression artérielle, compenser, sur la base de la valeur de compensation de pression d'air obtenue, la valeur de pression d'air détectée par le capteur de pression barométrique (5), et commander l'appareil d'entraînement (6) sur la base d'une valeur de pression d'air compensée ; et
l'appareil d'entraînement (6) est connecté électriquement à l'appareil d'alimentation et d'évacuation d'air (4) et est configuré pour entraîner, sous la commande du processeur (7), l'appareil d'alimentation et d'évacuation d'air (4) pour réaliser un gonflage ou un dégonflage,
dans lequel le dispositif de mesure de pression artérielle comprend en outre une cavité de trajet d'air (10), et la cavité de trajet d'air (10) est disposée dans la cavité (101) du corps (1) ; et
l'appareil d'alimentation et d'évacuation d'air (4) est connecté à la cavité de trajet d'air (10) via le premier trajet d'air (81), le capteur de pression barométrique (5) est connecté à la cavité de trajet d'air (10) via le deuxième trajet d'air (82), et la cavité de trajet d'air (10) est connectée à la cavité d'air de la poche gonflable (2) via un troisième trajet d'air (83).

2. Dispositif de mesure de pression artérielle selon la revendication 1, dans lequel le fait que le débitmètre (9) détecte une valeur de débit d'air entre l'appareil d'alimentation et d'évacuation d'air (4) et la poche gonflable (2) est spécifiquement :
le débitmètre (9) est situé dans le premier trajet d'air (81) et est configuré pour détecter une valeur de débit d'air dans le premier trajet d'air (81) ; et
le fait que le débitmètre (9) détecte une valeur de débit d'air entre le capteur de pression barométrique (5) et la poche gonflable (2) est spécifiquement :
le débitmètre (9) est situé dans le deuxième trajet d'air (82) et est configuré pour détecter une valeur de débit d'air dans le deuxième trajet d'air (82).

3. Dispositif de mesure de pression artérielle selon la revendication 1, dans lequel le débitmètre (9) est situé dans le troisième trajet d'air et est configuré pour détecter une valeur de débit d'air dans le troisième trajet d'air.

4. Dispositif de mesure de pression artérielle selon l'une quelconque des revendications 1 à 3, dans lequel le processeur (7) est en outre configuré pour :
commander le dispositif de mesure de pression artérielle pour réaliser un gonflage lorsque la poche gonflable (2) est retirée, de sorte que la valeur de débit d'air détectée par le débitmètre (9) change ;
obtenir une pluralité de valeurs de pression d'air détectées par le capteur de pression barométrique (5) lorsque le débitmètre (9) détecte une pluralité de valeurs de débit d'air différentes, et utiliser la pluralité de valeurs de pression d'air obtenues comme valeurs de compensation de pression d'air correspondant séparément à la pluralité de valeurs de débit d'air différentes ; et
établir la correspondance entre une valeur de débit d'air et une valeur de compensation de pression d'air sur la base de la pluralité de valeurs de débit d'air différentes et des valeurs de compensation de pression d'air correspondant séparément à la pluralité de valeurs de débit d'air différentes.

5. Dispositif de mesure de pression artérielle selon la revendication 4, dans lequel lors de l'établissement de la correspondance entre une valeur de débit d'air et une valeur de compensation de pression d'air sur la base de la pluralité de valeurs de débit d'air différentes et des valeurs de compensation de pression d'air correspondant séparément à la pluralité de valeurs de débit d'air différentes, le processeur (7) est spécifiquement configuré pour :
établir la correspondance entre une valeur de débit d'air et une valeur de compensation de pression d'air selon un procédé d'interpolation sur la base de la pluralité de valeurs de débit d'air différentes et des valeurs de compensation de pression d'air correspondant séparément à la pluralité de valeurs de débit d'air différentes.

6. Dispositif de mesure de pression artérielle, comprenant : un corps (1), un processeur (7), une poche gonflable (2), un appareil d'alimentation et d'évacuation d'air (4), un appareil d'entraînement (6), un premier capteur de pression barométrique (5a) et un second capteur de pression barométrique (5b), dans lequel
le corps (1) comprend une cavité (101), la cavité (101) est entourée d'une pluralité de parois latérales, et l'appareil d'alimentation et d'évacuation d'air (4), le premier capteur de pression barométrique (5a) et le second capteur de pression barométrique (5b) sont tous disposés dans la cavité (101) ;
la poche gonflable (2) a une cavité d'air, et la poche gonflable (2) est fixée à une extrémité du corps (1) ;
l'appareil d'alimentation et d'évacuation d'air (4) est connecté à la cavité d'air de la poche gonflable (2) via un premier trajet d'air (81) ;
le premier capteur de pression barométrique (5a) est connecté à la cavité d'air de la poche gonflable (2) via un deuxième trajet d'air (82) et est configuré pour détecter une valeur de pression d'air dans la cavité d'air ;
le second capteur de pression barométrique (5b) est configuré pour détecter une valeur de pression d'air dans la cavité (101) ; le processeur (7) est connecté électriquement au premier capteur de pression barométrique (5a), au second capteur de pression barométrique (5b) et à l'appareil d'entraînement (6), et est configuré pour : obtenir une valeur de compensation de pression d'air correspondante sur la base de la valeur de pression d'air détectée par le second capteur de pression barométrique (5b) et d'une correspondance entre une valeur de pression d'air de cavité et une valeur de compensation de pression d'air et qui est stockée dans le dispositif de mesure de pression artérielle, compenser, sur la base de la valeur de compensation de pression d'air obtenue, la valeur de pression d'air détectée par le premier capteur de pression barométrique (5a), et commander l'appareil d'entraînement (6) sur la base d'une valeur de pression d'air compensée ; et
l'appareil d'entraînement (6) est connecté électriquement à l'appareil d'alimentation et d'évacuation d'air (4) et est configuré pour entraîner, sous la commande du processeur (7), l'appareil d'alimentation et d'évacuation d'air (4) pour réaliser un gonflage ou un dégonflage,
dans lequel le dispositif de mesure de pression artérielle comprend en outre une cavité de trajet d'air (10), et la cavité de trajet d'air (10) est disposée dans la cavité (101) du corps (1) ; et
l'appareil d'alimentation et d'évacuation d'air (4) est connecté à la cavité de trajet d'air (10) via le premier trajet d'air (81), le premier capteur de pression barométrique (5a) est connecté à la cavité de trajet d'air (10) via le deuxième trajet d'air (82), et la cavité de trajet d'air (10) est connectée à la cavité d'air de la poche gonflable (2) via un troisième trajet d'air (83).

7. Dispositif de mesure de pression artérielle selon la revendication 6, dans lequel le second capteur de pression barométrique (5b) est disposé près de l'appareil d'alimentation et d'évacuation d'air (4).

8. Dispositif de mesure de pression artérielle selon l'une quelconque des revendications 6 ou 7, dans lequel le processeur (7) est en outre configuré pour :
commander le dispositif de mesure de pression artérielle pour réaliser un gonflage lorsque la poche gonflable (2) est retirée, de sorte qu'une première valeur de pression d'air détectée par le second capteur de pression barométrique (5b) change ;
obtenir une pluralité de secondes valeurs de pression d'air détectées par le premier capteur de pression barométrique (5a) lorsque le second capteur de pression barométrique (5b) détecte une pluralité de premières valeurs de pression d'air différentes, et utiliser la pluralité de secondes valeurs de pression d'air obtenues comme valeurs de compensation de pression d'air correspondant séparément à la pluralité de premières valeurs de pression d'air différentes ; et
établir la correspondance entre une valeur de pression d'air de cavité et une valeur de compensation de pression d'air sur la base de la pluralité de premières valeurs de pression d'air différentes et des valeurs de compensation de pression d'air correspondant séparément à la pluralité de premières valeurs de pression d'air différentes.

9. Dispositif de mesure de pression artérielle selon la revendication 8, dans lequel lors de l'établissement de la correspondance entre une valeur de pression d'air de cavité et une valeur de compensation de pression d'air sur la base de la pluralité de premières valeurs de pression d'air différentes et des valeurs de compensation de pression d'air correspondant séparément à la pluralité de premières valeurs de pression d'air différentes, le processeur (7) est spécifiquement configuré pour :
établir la correspondance entre une valeur de pression d'air de cavité et une valeur de compensation de pression d'air selon un procédé d'interpolation sur la base de la pluralité de premières valeurs de pression d'air différentes et des valeurs de compensation de pression d'air correspondant séparément à la pluralité de premières valeurs de pression d'air différentes.

10. Dispositif de mesure de pression artérielle, comprenant :
un corps (1), un processeur (7), une poche gonflable (2), un appareil d'alimentation et d'évacuation d'air (4), un appareil d'entraînement (6) et un capteur de pression barométrique (5), dans lequel
le corps (1) comprend une cavité (101), la cavité (101) est entourée d'une pluralité de parois latérales, et l'appareil d'alimentation et d'évacuation d'air (4) et le capteur de pression barométrique (5) sont tous deux disposés dans la cavité (101) ;
la poche gonflable (2) a une cavité d'air, et la poche gonflable (2) est fixée à une extrémité du corps (1) ;
l'appareil d'alimentation et d'évacuation d'air (4) est connecté à la cavité d'air de la poche gonflable (2) via un premier trajet d'air (81) ;
le capteur de pression barométrique est connecté à la cavité d'air de la poche gonflable (2) via un deuxième trajet d'air (82) et est configuré pour détecter une valeur de pression d'air dans la cavité d'air ;
le processeur (7) est connecté électriquement au capteur de pression barométrique (5) et à l'appareil d'entraînement (6), et est configuré pour : obtenir une valeur de compensation de pression d'air correspondante sur la base d'un état d'entraînement de l'appareil d'entraînement (6) et d'une correspondance entre un état d'entraînement et une valeur de compensation de pression d'air et qui est stockée dans le dispositif de mesure de pression artérielle, compenser, sur la base de la valeur de compensation de pression d'air obtenue, une valeur de pression d'air détectée par le capteur de pression barométrique (5), et commander l'appareil d'entraînement (6) sur la base d'une valeur de pression d'air compensée ; et
l'appareil d'entraînement (6) est connecté électriquement à l'appareil d'alimentation et d'évacuation d'air (4) et est configuré pour entraîner, sous la commande du processeur (7), l'appareil d'alimentation et d'évacuation d'air (4) pour réaliser un gonflage ou un dégonflage,
dans lequel le dispositif de mesure de pression artérielle comprend en outre une cavité de trajet d'air (10), et la cavité de trajet d'air (10) est disposée dans la cavité (101) du corps (1) ; et
l'appareil d'alimentation et d'évacuation d'air (4) est connecté à la cavité de trajet d'air (10) via le premier trajet d'air (81), le capteur de pression barométrique (5) est connecté à la cavité de trajet d'air (10) via le deuxième trajet d'air (82), et la cavité de trajet d'air (10) est connectée à la cavité d'air de la poche gonflable (2) via un troisième trajet d'air (83).

11. Dispositif de mesure de pression artérielle selon la revendication 10, dans lequel le processeur (7) est en outre configuré pour :
commander le dispositif de mesure de pression artérielle pour réaliser un gonflage dans une pluralité d'états d'entraînement différents lorsque la poche gonflable (2) est retirée ;
obtenir une pluralité de valeurs de pression d'air détectées par le capteur de pression barométrique (5) lorsque le dispositif de mesure de pression artérielle réalise un gonflage dans la pluralité d'états d'entraînement différents, et utiliser la pluralité de valeurs de pression d'air obtenues comme valeurs de compensation de pression d'air correspondant séparément à la pluralité d'états d'entraînement différents ; et
établir la correspondance entre un état d'entraînement et une valeur de compensation de pression d'air sur la base de la pluralité d'états d'entraînement différents et des valeurs de compensation de pression d'air correspondant séparément à la pluralité d'états d'entraînement différents.

12. Dispositif de mesure de pression artérielle selon la revendication 11, dans lequel lors de l'établissement de la correspondance entre un état d'entraînement et une valeur de compensation de pression d'air sur la base de la pluralité d'états d'entraînement différents et des valeurs de compensation de pression d'air correspondant séparément à la pluralité d'états d'entraînement différents, le processeur (7) est spécifiquement configuré pour :
établir la correspondance entre un état d'entraînement et une valeur de compensation de pression d'air selon un procédé d'interpolation sur la base de la pluralité d'états d'entraînement différents et des valeurs de compensation de pression d'air correspondant séparément à la pluralité d'états d'entraînement différents.

13. Dispositif électronique, comprenant le dispositif de mesure de pression artérielle selon l'une quelconque des revendications 1 à 12.
